# EUROPÄISCHE PATENTANMELDUNG

(11) **EP 4 501 330 A2**
(43) Veröffentlichungstag der Anmeldung: **05.02.2025**
(21) Anmeldenummer: 24208093.5
(22) Anmeldetag: 19.05.2017
(51) Int. Cl.: A61K 31/59

(54) **PHARMAZEUTISCHE ZUSAMMENSETZUNGEN MIT ANTI-RANKL-ANTIKÖRPERN, KALZIUM UND VITAMIN D, GEEIGNET ZUR BEHANDLUNG UND/ODER PROPHYLAXE VON ERKRANKUNGEN DES KNOCHENSTOFFWECHSELS UND VON THERAPIEBEDINGTEN NEBENWIRKUNGEN WIE HYPOKALZÄMIEN**

(30) Priorität: 20.05.2016 DE 102016006557
(62) Teilanmeldung aus: 17728455.1
(71) Anmelder: Karl, Christoph, 8274 Tägerwilen (CH)
(72) Erfinder: Karl, Christoph, 8280 Kreuzlingen (CH); Thierolf, Rüdiger, 64832 Babenhausen (DE)
(74) Vertreter: Sommer, Andrea

(57) **Zusammenfassung**

Die vorliegende Erfindung beinhaltet pharmazeutische Zusammensetzungen, umfassend Anti-RANKL-Antikörper, Kalzium und Vitamin D, die zur Behandlung und/oder Prophylaxe von Erkrankungen des Knochenstoffwechsels und von therapiebedingten Nebenwirkungen wie Hypokalzämien verursacht durch die Wirkung der Anti-RANKL-Antikörper, geeignet sind.

## Beschreibung

Pharmazeutische Zusammensetzungen mit Anti-RANKL-Antikörpern, Kalzium und Vitamin D, geeignet zur Behandlung und/oder Prophylaxe von Erkrankungen des Knochenstoffwechsels und von therapiebedingten Nebenwirkungen wie Hypokalzämien

### Technisches Gebiet

Die vorliegende Erfindung beinhaltet pharmazeutische Zusammensetzungen, umfassend Anti-RANKL-Antikörper, Kalzium und Vitamin D, die zur Behandlung und/oder Prophylaxe von Erkrankungen des Knochenstoffwechsels und von therapiebedingten Nebenwirkungen wie Hypokalzämien verursacht durch die Wirkung der Anti-RANKl-Antikörper, geeignet sind.

### Beschreibung

Die Behandlung von Erkrankungen des Knochenstoffwechsels wie der Osteoporose, Tumorerkrankungen inklusive induziertem Knochenverlust (CTIBL, cancer treatmentinduced bone loss), Morbus Paget, oder der Hypokalzämie bilden einen bedeutenden Schwerpunkt in der medizinischen Forschung. Der menschliche Knochen besteht aus einer organischen Matrix, die mit anorganischen Salzen - vorwiegend Kalzium und Phosphat in Form von Hydroxylapatit (Ca₃(PO₄)₂ • Ca(OH)₂) kombiniert ist. Als Grundprinzip liegt der Knochenphysiologie beim Menschen ein ständiger Auf- und Abbau (bone turnover) von Knochensubstanz zugrunde. Knochen AUF- und AB-Bau im Rahmen des fortwährenden Remodellings ist dabei hormonell (im Wesentlichen durch Östrogen, Parathormon (PTH)) und durch Signaltransduktionswege (im Wesentlichen RANKL ("receptor activator of nuclear factor-kB ligand")/OPG (Osteoprotegerin) und Ca/PTH/Vitamin D3) reguliert. Während in der Kindheit und v.a. der Pubertät mehr Knochen auf- als abgebaut wird, kommt es im Alter, bei Frauen insbesondere während und nach der Menopause, zunehmend zum 'netto'-Knochenabbau.

Im Laufe des menschlichen Lebens ist Knochen fortwährend physiologischen mechanischen Belastungen ausgesetzt und passt sich entsprechend an. Erstmals beschrieben wurde dies von Julius Wolff 1862 und das Wolffsche Gesetz stellt die Grundlage für das biomechanische Verständnis des Knochens dar. Wolff konnte bei der Untersuchung von Femurköpfen zeigen, dass der Knochen sich in seiner Form an die Funktion anpasst und bei dauerhafter Entlastung degeneriert. So wird im Rahmen des "Remodelling" nicht belasteter Knochen abgebaut, was in den ,Bed-rest'-Studien mit absoluter Bettruhe über einen Zeitraum von 60 Tagen untersucht wurde. Mechanisch stark belasteter Knochen wird hingegen entsprechend der Kraftlinien verstärkt, was beispielhaft an Unterarmknochen von Tennisspielern gezeigt werden konnte.

Neben regelmäßigen Umbauvorgängen kommt es bei Krafteinwirkung auf den Knochen zu Mikroschäden oder Kontinuitätsunterbrechungen (Frakturen) mit und ohne Defektbildung, die narbenlos ausheilen können. Bei anorganischen Materialien führen repetitive Belastungen unterhalb der Bruchgrenze zur Materialermüdung/zum Materialermüdungsbruch, indem kleine Risse entstehen und wachsen, bis sie eine kritische Größe erreichen und das Material bricht. Als quasi-sprödes Material, das durch Ausbildung von kleinen Rissen (sogenannte "micro-cracks") Energie absorbiert, kann es Knochenfrakturen vermeiden. "Micro-cracks" wurden bereits 1960 von Harold Frost beschrieben und sind klar begrenzte Risse von 50-100µm Länge, die vornehmlich im interstitiellen Knochen auftreten. Sie sind in der Regel entsprechend der biomechanischen Belastung in der longitudinalen Achse länger als in der transversalen Achse, entstehen bereits bei physiologischen, repetitiven Belastungen wie Gehen und Laufen im trabekulären wie kortikalen Knochen und treten signifikant vermehrt im Alter auf.

Im Knochen bleiben "micro-cracks" in der Regel klinisch inapparent, da sie fortwährend im Rahmen des "Remodelling" repariert und geheilt werden. Durch den Riss kommt es zur Apoptose von Osteozyten, welche Faktoren freisetzen, u.a. RANKL. Diese stoßen die osteoklastäre Resorption an, mit konsekutivem osteoblastärem Knochenanbau.

Während gesunder Knochen das Fortschreiten von Mikroschäden verhindert und repariert, kann es im gealterten Knochen oder unter z.B. Suppression des "Remodellings" durch hoch potente antiresorptive Medikamente wie beispielsweise Bisphosphonate oder Anti-RANKL-Antikörpern zur Akkumulation und gegebenenfalls erhöhter Bruchanfälligkeit kommen, z.B. bei atypischen Femurfrakturen.

Bei den fortwährend im Rahmen des Remodellings stattfindenden Umbauvorgängen am Knochen sind im Wesentlichen Osteoblasten, Osteoklasten und Osteozyten involviert. Während Osteoklasten den Knochen abbauen, können Osteoblasten den Knochen anbauen.

Die Rolle der Osteozyten ist letztlich bis heute noch nicht bis ins Detail geklärt. Es handelt sich dabei um im Knochen eingemauerte terminal differenzierte Zellen osteoblastären Ursprungs, welche untereinander verbunden sind und kommunizieren. Kommt es zu Verletzungen der Osteozyten-Ausläufer, z.B. im Rahmen der oben beschriebenen Microcracks, wird zunächst die osteoklastäre Resorption initiiert, auf welche dann wiederum der osteoblastäre Knochenanbau folgt. Die Interaktion zwischen Osteoblasten, Osteoklasten und Osteozyten ist im Wesentlichen durch den RANKL/OPG-Signaltransduktionsweg (Differenzierung von Osteoklasten) und den WNT/DKK/SOST (WNT ist ein Signalprotein, welches sich aus dem Wingless (Wg) und Int-1-Protein zusammensetzt; DKK=Dickkopf; SOST=Symbol für das Protein Sclerostin))-Pathway (Differenzierung der Osteoblasten) gesteuert.

Beim Knochenanbau sind vor allem der WNT-Pathway zur Differenzierung der Osteoblasten als auch der PTH-Signaltransduktionsweg involviert. Die Differenzierung von hämatopoetischen Stammzellen zu Osteoklasten wird über den RANKL/OPG-Signaltransduktionsweg gesteuert.

Der Knochen dient als Kalziumspeicher für den menschlichen Körper und ist somit entscheidend an der Kalzium-Phosphat-Homöostase beteiligt. Durch Knochenauf und - abbau können überschüssige Kalziumreserven aus dem Blut gespeichert oder auch im Bedarfsfall wieder zur Verfügung gestellt werden.

Bei diversen Erkrankungen des Knochenstoffwechsels ist dieses empfindliche Gleichgewicht gestört. Die häufigste Erkrankung des Knochenstoffwechsels ist die Osteoporose. Erkrankungen, welche den Knochenstoffwechsel beeinflussen, sind ferner Morbus Paget, Osteogenesis imperfecta, primäre Knochentumore wie das multiple Myelom (Plasmozytom), Osteosarkom und Knochenmetastasen. Bei Komplikationen durch Knochenmetastasen/Knochentumore spricht man im englischen Sprachgebrauch von "skeletal related events" (SRE oder "skelettbezogene Komplikationen"). Der Begriff "skelettbezogene Komplikationen" (SRE) beinhaltet akute Ereignisse wie pathologische Knochenfrakturen, Rückenmarkskompressionen, Knochenschmerzen oder eine tumorinduzierte Hyperkalzämie sowie therapeutische Interventionen wie Knochenbestrahlung oder operative Eingriffe am Knochen, die zum Beispiel bei Patienten mit Knochenmetastasen oder primären Knochentumoren auftreten können.

Osteoporose bewirkt eine Abnahme der Knochendichte, die zusammen mit der verringerten Knochenqualität zu einem vermehrten Knochenbruchrisiko führt. Als Osteoporose wird derzeit eine messbare Knochendichteminderung kleiner -2,5 Standardabweichungen (sogenannter T-Wert oder englisch T-score) von einem gesunden Kollektiv (Frauen, 30 Jahre), gemessen mittels DXA (dual-energy X-ray absorptiometry) von der WHO definiert. Durch einen stattgefundenen Knochenbruch spricht man von einer manifesten Osteoporose. Osteoporose ist somit eine systemische Erkrankung des Knochens, die sich häufig durch eine Fraktur bei inadäquatem Trauma manifestiert. In Deutschland sind ca. 6 Millionen Menschen von der Volkskrankheit Osteoporose betroffen und jährlich erleiden Osteoporosepatienten mehr als 720.000 Frakturen. Die postmenopausale Osteoporose ist die weitaus häufigste Ursache bei vertebralen und nicht-vertebralen Fragilitätsfrakturen. Das Risiko eine Fraktur zu erleiden steigt mit zunehmendem Alter erheblich an.

Bereits in den ersten 5 Jahren nach der Menopause wird mit ausbleibender Östrogenproduktion ein individuell unterschiedlich starker Abbau der Knochenmasse beobachtet. Dabei kann der postmenopausale Knochenmasseverlust bis zu 15% pro Jahr betragen und führt bei Frauen mit geringer Spitzenknochenmasse sehr schnell zur ersten osteoporotischen Fraktur.

Mangelhafte Kalziumzufuhr sowie ein dezimierter Vitamin D-Spiegel im Blut führen in der Bilanz zu einem Kalziumabbau im Knochen. Es ist außerdem zu erwarten, dass mit zunehmendenm Alter weitere Pathomechanismen hinzukommen, die direkt oder indirekt den Knochenabbau fördern. Bis zum 65. Lebensjahr hat sich zum Beispiel die Fähigkeit Kalziumionen aus der Nahrung aufzunehmen um etwa 50% reduziert (im Vergleich zu Jugendlichen). Altersbedingte Begleiterkrankungen, wie Diabetes mellitus, eingeschränkte Nierenfunktion, Immobilität oder eine Therapie mit Glitazonen, sind nur einige Beispiele aus der langen Liste an Risikofaktoren, die nachweislich einen additiven Effekt auf den Knochenabbau haben und damit ein latentes Fortschreiten der Osteoporose fördern.

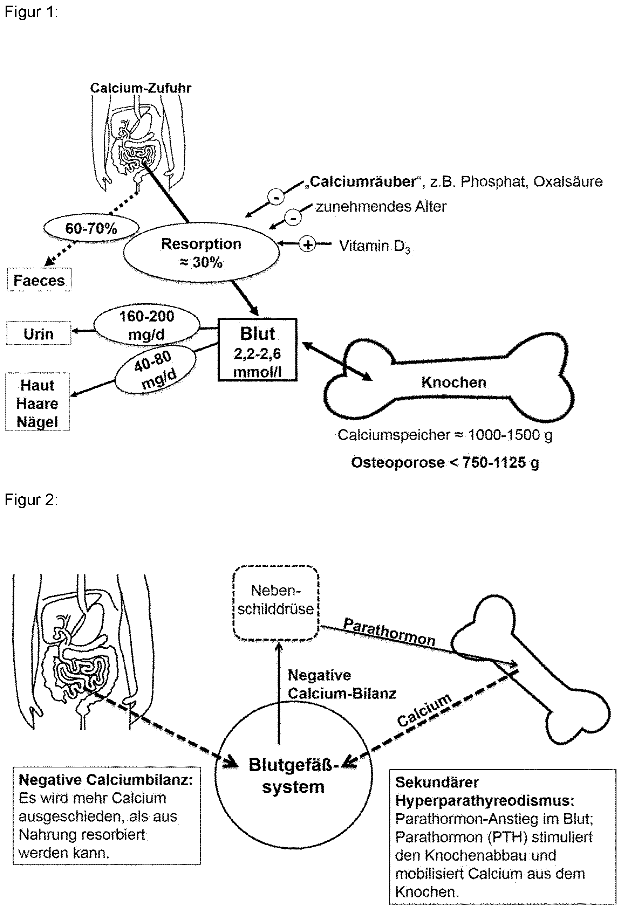

Die Figur 1 verdeutlicht, dass etwa nur ein Drittel der im Verdauungstrakt angebotenen Kalziummenge resorbiert wird. 65-70% des Nahrungskalziums wird mit dem Stuhl wieder ausgeschieden. Der Resorptionsvorgang selbst kann zusätzlich durch verschiedene Faktoren positiv oder negativ beeinflusst werden. Zum Beispiel Nahrungsmittel, die sehr stark mit Phosphaten angereichert sind, können die Aufnahme von Kalzium extrem behindern.

Zu den Hormonen, die die Kalzium-Homöostase steuern, zählen neben den Sexualhormonen (Östrogen, Testosteron) v.a. Calcitonin, Parathormon (Parathyrin) und Calcitriol (aktive Form von Vitamin D3). Calcitriol ist essentiell, um die Absorption von Kalzium und Phosphat im Dünndarm zu ermöglichen. Außerdem steigert Calcitriol die Rückresorption von Kalzium in den Nieren und stimuliert die Knochenmineralisation (Einbau von Kalzium in die Knochenmatrix).

Beispielsweise entsteht eine negative Kalziumbilanz, wenn der Körper mehr Kalzium ausscheidet, als er über den Darm resorbieren kann. Sollte dieser Zustand über längere Zeit bestehen bleiben, kommt es zum verstärkten Knochenabbau und einem sekundären Hyperparathyreodismus. Um das "fehlende" Kalzium aus dem Knochen zu moblisieren, wird in der Nebenschilddrüse "Parathormon" gebildet. Wie in Figur 2 dargestellt, stimuliert Parathormon den Knochenabbau und führt auf diese Weise zu einem Anstieg der Kalziumkonzentration im Blut. Die Kalziumkonzentration verbleibt im Blut somit relativ konstant zu Lasten des Knochens, der bei vorliegendem Kalziummangel für eine Erhöhung von Kalzium im Blut sein Kalzium spendet.

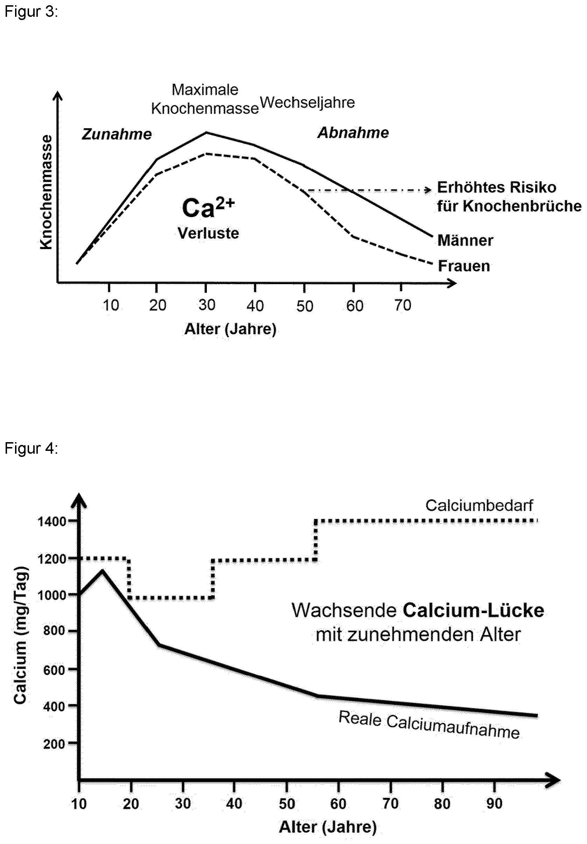

Figur 3 zeigt eine Darstellung über die "Kalziumverluste im Alter": Bereits nach dem 40. Lebensjahr beginnt ein schleichender Knochendichteverlust bei Männern und Frauen von 2-3% pro Jahr. Zu einer enormen Steigerung der jährlichen Verluste kann es bei mehr als 20% der Frauen nach der Menopause kommen. Oftmals unbemerkt verlieren die betroffenen Frauen mehr als ein Viertel ihrer Knochenmasse (=Kalzium) in einem relativ kurzen Zeitraum und haben dadurch ein sehr hohes Risiko für Frakturen im Laufe ihres weiteren Lebens. Die Erkrankung "Osteoporose" wird in vielen Fällen zu spät diagnostiziert - meistens erst dann, wenn bereits eine osteoporotische Fraktur (d.h. ohne traumatisches Ereignis) zum Beispiel an Wirbelkörper, Hüft- oder Handgelenk aufgetreten ist.

Ein nicht zu unterschätzendes und zusätzliches Problem der betroffenen Männer und Frauen im Alter ab 50 Jahren ist die wachsende Kalzium-Lücke, die sich aus einer progredienten negativen Kalziumbilanz ergibt. Wie in Figur 4 dargestellt ergibt sich im Alter eine zunehmende Diskrepanz zwischen dem täglichen Kalziumbedarf und der realen Aufnahme von Kalzium aus dem Gastrointestinaltrakt. Der altersbedingte hohe Bedarf an Kalzium wird im Wesentlichen durch folgende Faktoren beeinflusst: nachlassende Kalziumresorption im Glastrointestinaltrakt, kalziumarme Ernährung (wenig Milchprodukte), Vitamin D Mangel, wenig Bewegung, Kalziumspeicher "Knochen" soll wieder aufgefüllt werden (Aufbau neuer Knochenmasse), erhöhtes Parathormon im Blut (erhöhter Knochenabbau und Kalziumverluste) oder eingeschränkte Nierenfunktion (verminderte Kalzium-Rückresorption und weniger aktives Vitamin D).

Zum Ausgleich der natürlichen Kalziumverluste über Haut und Nieren sowie zur lebensnotwendigen Aufrechterhaltung eines konstanten Kalziumspiegels im Blut hat der menschliche Organismus letztendlich nur 2 Möglichkeiten:
1.) Kalzium aus dem "Kalziumspeicher Knochen" mobilisieren. Nachteil: Der dafür notwendige Abbau der Knochensubstanz kann langfristig zu Stabilitätsverlusten und Frakturen führen.
2.) Ausreichende Resorption von Kalzium aus dem Gastrointestinaltrakt und RückResorption von Kalzium in den Nieren. Beides wird durch Vitamin D gesteuert.

Die biologisch aktive Form des Vitamins D3 (1α,25-Dihydroxycholecalciferol; Calcitriol) fördert die Aufnahme von Kalzium aus dem Gastrointestinaltrakt ins Blut. Die maximale Serumkonzentration wird innerhalb von drei bis sechs Stunden nach der Aufnahme erreicht. In der Summe führt Vitamin D zu einer Förderung der Aufnahme von Kalzium in den Körper. Eine Überdosierung kann zu einer Vitamin D-Vergiftung führen, da der Körper Vitamin D speichert. Die Vitamin D-Vergiftung kann zu einer starken Demineralisierung des Knochens führen, welche zu Frakturen führt. Gleichzeitig können hohe Kalziumserumkonzentrationen zu einer anormalen Kalzifizierung der verschiedensten weichen Gewebe führen. Zusätzlich können aufgrund der erhöhten renalen Kalziumausscheidung Nierensteine entstehen.

Ein Vitamin D-Mangel führt aufgrund einer verminderten Resorption aus dem Darm dazu, dass weniger Kalzium aus der Nahrung ins Blut gelangt.

Ein Kalzium- und/oder Vitamin D-Mangel wird durch die Supplementation mit Kalzium- und Vitamin D-haltigen Präparaten behandelt. Die Anwendung von Kalzium und Vitamin D ist jedoch nicht unumstritten. Weltweit werden Diskussionen zur Verträglichkeit von Kalzium geführt und einige Wissenschaftler sehen in der Kalziumsupplementation ein kardiovaskuläres Risiko. Es wird behauptet, Kalzium erhöhe das Risiko für kardiovaskuläre Ereignisse wie Herzinfarkt. In Deutschland hat diese Diskussion dazu geführt, dass die Kalziumsupplementation häufig unterbleibt.

Für die Osteoporose bedeutet der Kalziummangel eine Verstärkung des Krankheitsbildes mit einer Erhöhung des Frakturrisikos.

Bei der manifesten Osteoporose ist der Knochenmineralgehalt vermindert (T-Score: <-2,5) und es haben sich Frakturen ereignet, beispielsweise 1 bis 3 Wirbelkörperfrakturen.

Eine Glukocorticoid-Langzeittherapie kann zu einer schweren glukocorticoid- oder corticoid-induzierten Osteoporose mit Frakturen führen. Bei einer Gabe von >5 mg Prednison-Äquivalent pro Tag über >3 Monate werden präventive bzw. therapeutische Maßnahmen empfohlen. Vor allem in den ersten sechs Monaten nach Therapiebeginn, bei erheblichen Dosissteigerungen im Verlauf und bei hochdosierter Langzeittherapie ist mit einer signifikanten Knochendichteabnahme zu rechnen, was in der Behandlung zu berücksichtigen ist. Der glukocorticoidinduzierte Knochenverlust hat mehrere Ursachen. Glukocorticoide führen am Beginn einer Therapie zu einer Steigerung der Knochenresorption. Steroide hemmen die Proliferation und Funktion von Osteoblasten und steigern deren Apoptose. Sie bewirken dadurch eine verminderte Knochenneubildung. Sie führen gleichzeitig zu einer negativen Kalziumbilanz durch Hemmung der intestinalen Kalziumresorption und Steigerung der Harn-Kalzium-Ausscheidung.

Eine weitere Erkrankung des Knochenstoffwechsels ist Morbus Paget, ebenfalls bekannt als Osteodystrophia deformans, Ostitis deformans, Paget-Syndrom oder Paget-Krankheit mit derzeit unbekannter Ursache, wobei sich genetische, virale und Umwelteinflüsse in der Diskussion für eine mögliche Ursache befinden. Morbus Paget ist eine chronische Erkrankung des Knochenstoffwechsels, charakterisiert durch lokal erhöhte Knochenumbauvorgänge mit dem Risiko von Verformungen, chronischen Schmerzen und Frakturen sowie artikulären, neurologischen, und kardiologischen Komplikationen.

Solide primäre Tumore wie beispielsweise Mamma-, Prostata-, Lungen-, Darm- oder Knochenkarzinom (z.B. Osteosarkom) können sich im Knochen manifestieren und somit Knochenmetastasen bzw. Knochenerkrankungen hervorrufen. Knochenmetastasen sind Absiedlungen anderer Tumore in die Knochen und können dort zu Schmerzen und Brüchen führen. Verantwortlich ist ein verstärkter Knochenabbau oder eine überschießende Produktion von minderwertiger Knochensubstanz. Das verringert die Stabilität der Knochen. Tumorbedingte Knochenkomplikationen zeigen eine hohe Morbidität, eine erhöhte Knochenbruchrate, Nervenkompressionen und teilweise unerträgliche Schmerzen. Wird vermehrt Kalzium aus dem Knochen freigesetzt und ins Blut abgegeben, kann sich eine tumorinduzierte Hyperkalzämie entwickeln. So ist beispielsweise der Anteil an Patienten mit moderaten oder starken Knochenschmerzen (≥ 4 Punkte im BPI-SF [brief pain inventory, short form]) mit metastasiertem Mamma-, Prostata-, Lungen-, Darm- oder Knochenkarzinom (z.B. Osteosarkom) mit über 80%, gefolgt erst von neuralgischen Schmerzen mit ca. 10%, mit Abstand am höchsten (Cleeland, C.S. et al., Ann. Onc. 2005, 16: 972-980).

Der Begriff "Multiples Myelom" bzw. "Plasmozytom" oder "Kahler-Krankheit" beinhaltet eine Krebserkrankung des Knochenmarks, bei der die antikörperproduzierenden Zellen (Plasmazellen) stark vermehrt werden. Diese malignen Plasmazellen vermehren sich unkontrolliert und bilden funktionslose Antikörper oder Teile davon. Der Krankheitsverlauf kann sehr unterschiedlich sein mit moderaten bis zu hochmalignen Verläufen, die ohne Behandlung schnell zum Tod des Patienten führen. Die Symptome werden durch das Wachstum der Zellen verursacht oder durch die produzierten Antikörper bzw. deren Bruchstücken, die sich in Knochenschmerzen, Verringerung der Knochenmasse und in Frakturen manifestieren können, einhergehend mit einer erhöhten Freisetzung von Kalzium ins Blut, die zu tumor-induzierter Hyperkalzämie führen kann. Die Anzahl der Leukozyten nimmt ab, während sich die große Anzahl an Antikörpern im Gewebe ablagert und zu Funktionsstörungen vieler Organe, zu Nierenversagen und zur Beeinträchtigung der Durchblutung führt.

Arzneimittel zur Behandlung von Erkrankungen des Knochenstoffwechsels sind bekannt. Bei der medikamentösen Therapie der Osteoporose geht es in erster Linie darum den pathologischen Knochenmasseverlust zu stoppen. Die negative Bilanz (Ungleichgewicht zwischen An- und Abbau von Knochensubstanz) kann entweder antiresorptiv, durch eine Hemmung der Osteoklasten (Knochenabbau) oder anabol, durch eine Stimulation der Osteoblasten (Knochenaufbau) ausgeglichen werden. Erst wenn An- und Abbauprozesse wieder im Gleichgewicht zueinander stehen und eine ausreichende Versorgung mit Kalzium und Vitamin D gewährleistet wird, kann sich neue Knochenmasse aufbauen und das Risiko für osteoporotische Frakturen reduziert werden.

Zur Therapie der Osteoporose kann einerseits der osteoklastäre Knochenabbau durch Anti-RANKL-Antikörper gehemmt, andererseits durch anabole Medikamente der Knochenanbau stimuliert werden. Hier ist das rekombinante 1-34 Fragment des Parathormons zur Therapie der Osteoporose zugelassen.

Antiresorptiva wie Anti-RANKL-Antikörper sind derzeit Standard zur Behandlung metabolischer Knochenerkrankungen, die eine effektive Hemmung des Knochenabbaus erforderlich machen. Hierzu zählt der Einsatz dieser Substanzklassen zur Behandlung der Osteoporose wie auch -mit deutlich höheren Dosen und kürzeren Behandlungsintervallenzur Behnadlung und/oder Prävention von tumorbedingten Skelettkomplikationen.

Anti-RANKl-Antikörper bzw. deren Derivate werden üblicherweise verwendet, um die oben genannten Erkrankungen wie Osteoporose oder tumorbedingte Knochenerkrankungen zu behandeln.

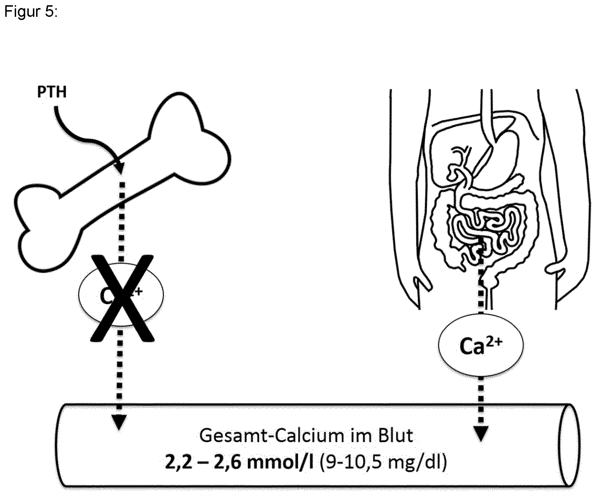

Als Folge der Anwendung von Anti-RAN KL-Antikörpern, verbleibt das Kalzium im Knochen. Eine Stimulation des Knochenabbaus z.B. durch Parathormon wird dadurch mehr oder weniger stark verhindert. Es entsteht eine Hypokalzämie-Gefahr. Eine Hypokalzämie liegt vor, wenn das Gesamtkalzium im Blutserum unter 2,2 mmol/l (9 mg/dl) liegt. Durch die medikamentöse Hemmung der Osteoklasten kann die Kalziumkonzentration im Blut nur noch über eine Zufuhr von Kalzium von außen (oral oder intravenös) ausgeglichen werden. Dies ist in Figur 5 dargestellt. PTH aktiviert die Freisetzung von Kalzium aus dem Knochen, die allerdings durch die Hemmung der Osteoklasten aufgrund der Anwendung der Anti-RANKL-Antikörper verhindert wird. Daher ist die Zufuhr von Kalzium von außen essentiell.

Die Hypokalzämie-Gefahr wird noch deutlich verstärkt, wenn ein sekundärer Hyperparathyreoidismus bei Vitamin D-Mangel vorliegt. Bei diesem häufigen Vitamin D-Mangelzustand wird zum Ausgleich des Serum-Kalziumspiegels vermehrt Parathormon ausgeschüttet. Parathormon erhöht indirekt die Kalziumkonzentration im Blutplasma durch Aktivierung der Osteoklasten.

Durch die antiresorptive Therapie wird die Hypokalzämie verstärkt, da die Osteoklasten gehemmt werden.

Insbesondere in der onkologischen Anwendung von Antiresorptiva wie Anti-RANKL-Antikörpern kann sich eine Unterversorgung mit Kalzium und/oder Vitamin D auf dramatische Weise zuspitzen. Aufgrund der hohen antiresorptiven Potenz erhielt sowohl PROLIA (60mg Denosumab) als auch XGEVA (120mg Denosumab) im September 2014 einen sogenannten "Rote-Hand-Brief" mit dem ausdrücklichen Hinweis für Ärzte: "Eine Ergänzung mit Calcium und Vitamin D ist bei allen Patienten erforderlich, außer bei bestehender Hyperkalzämie."

Auf diese Weise verursachte Hypokalzämien können zu Herzrhythmusstörungen und bei besonders schweren Verläufen sogar zum Tod führen, wenn die Hypokalzämie nicht rechtzeitig entdeckt und behandelt wird. Hypokalzämien bleiben zudem vielfach unentdeckt, da der Kalziumspiegel in der ambulanten klinischen Behandlung nicht in engen Zeitabständen als Laborparameter gemessen wird. Um die therapiebedingte Nebenwirkung der Hypokalzämie abzuwenden, wird vorliegend eine Supplementation mit Kalzium und Vitamin D bei der Anwendung von Anti-RANKL-Antikörpern bereitgestellt, die eine Hypokalzämie verhindern kann.

Eine unbeachtete Unterversorgung mit Kalzium und Vitamin D kann zudem den gesamten Erfolg einer antiresorptiven Therapie z.B. mit Anti-RANKL-Antikörpern negativ beeinflussen und somit den gewünschten Schutz vor neuen Frakturen reduzieren bzw. die Anzahl der Frakturen sogar erhöhen, wenn die Kalzium und Vitamin D-Supplementation unter antiresorptiver Therapie unterlassen wird.

Als Nebenwirkung von Anti-RANKL-Antikörpern werden Kieferosteonekrosen aufgeführt. Kieferosteonekrosen können der Behandlung mit Anti-RANKL-Antikörpern auftreten und zu Schmerzen im Mund, nicht heilenden Wunden bis zur Auflösung des Kiefers führen.

Als weitere Nebenwirkungen einer antiresorptiven Therapie können kardiovaskuläre Nebenwirkungen wie Herzrhythmusstörungen, Krämpfe sowie ein sekundärer Hyperparathyreoidismus auftreten.

Die vorliegende Erfindung löst diese Probleme und stellt neue pharmazeutische Zusammensetzungen von Anti-RANKL-Antikörpern und Kalzium und Vitamin D und/oder deren Derivaten bereit, um die Erkrankungen des Knochenstoffwechsels besser zu behandeln bzw. diesen Erkrankungen vorzubeugen und auf diese Weise die optimale Wirkung sicherzustellen, sowie therapiebedingte Nebenwirkungen wie die Hypokalzämie und/oder unsachgemäßen Gebrauch zu verhindern oder letzteren vorzubeugen.

### Zusammenfassung der Erfindung

In einem ersten Aspekt betrifft die Erfindung die Verwendung von pharmazeutischen Zusammensetzungen umfassend Anti-RANKL-Antikörpern und/oder deren antigenbindende Fragmente und Kalzium und Vitamin D zur Behandlung von Osteoporose, postmenopausaler Osteoporose, manifester Osteoporose, corticoinduzierter Osteoporose, Osteoporose des Mannes oder der Frau, Morbus Paget, Osteogenesis imperfecta zur Prophylaxe von Frakturen der vorgenannten Erkrankungen, sowie zur Verwendung in der Behandlung und/oder Prophylaxe der durch Anti-RANKL-Antikörper therapiebedingten Hypokalzämie, dadurch gekennzeichnet, dass die pharmazeutische Zusammensetzung umfassend Anti-RANKL-Antikörper und/oder deren antigenbindende Fragmente werden, stärker bevorzugt in einer Dosierung von 30 bis 90 mg in 1-2 ml Lösung (20-60 mg/ml), stärker bevorzugt 50-70 mg, noch stärker bevorzugt 60mg, bevorzugt in 1 ml Lösung (60 mg/ml) subkutan jährlich oder halbjährlich und die pharmazeutische(n) Zusammensetzung(en) umfassend Kalzium mit einer Dosierung von 400-600mg pro Tag, besonders bevorzugt 500mg pro Tag oral verabreicht wird und Vitamin D in einer Dosierung von 800-1200 I.E. (Internationale Einheiten) Vitamin D, besonders bevorzugt 1000 I.E. Vitamin D täglich oral verabreicht wird.

In einem weiteren Ausführungsbeispiel betrifft die Erfindung die Verwendung von pharmazeutischen Zusammensetzungen umfassend Anti-RANKL-Antikörper und/oder deren antigenbindende Fragmente und Kalzium und Vitamin D zur Prävention skelettbezogener Komplikationen, insbesondere pathologischen Frakturen, Bestrahlung des Knochens, Rückenmarkskompression, operativen Eingriffen am Knochen, Knochenmetastasen, Schmerzen bei Knochenmetastasen, Nerveneinklemmungen oder Deformierungen aufgrund eines oder mehrerer solider Tumore wie beispielsweise Brustkrebs, Prostatakrebs, Lungenkrebs oder multiplem Myelom, sowie der Prophylaxe von Frakturen bei den vorgenannten Erkrankungen und zur Verwendung in der Behandlung und/oder Prophylaxe der durch Anti-RANKL-Antikörper therapieinduzierten Hypokalzämie, dadurch gekennzeichnet, dass Anti-RANKL-Antikörper und/oder deren antigenbindende Fragmente verabreicht werden, bevorzugt in einer Dosierung von 60-180 mg in 1-3 ml Lösung, stärker bevorzugt 80-150 mg, noch stärker bevorzugt 120 mg, insbesondere in 1,7 ml Lösung (70 mg/ml) Anti-RANKL-Antikörper subkutan zur mindestens 3-4 wöchigen Anwendung und 400-600mg Kalzium täglich, besonders bevorzugt 500mg Kalzium täglich und 800-1200 I.E. Vitamin D täglich, besonders bevorzugt 1000 I.E. Vitamin D täglich oral verabreicht werden.

Ferner betrifft die Erfindung in weiteren Aspekten die Verwendung von verschiedenen Kalziumverbindungen und Vitamin D zur Verwendung in der Behandlung und/oder Prophylaxe von therapiebedingten Nebenwirkungen, in den vorgenannten Ausführungsbeispielen, sowie pharmazeutisch geeignete Hilfsstoffe und Lösungsmittel.

### Ausführliche Beschreibung der Erfindung

Anti-RANKL-Antikörper sind sehr wirksame Antiresorptiva und werden zur Behandlung von Erkrankungen des Knochenstoffwechsels angewendet. Trotz ihrer Wirksamkeit ist die Behandlung mit Anti-RANKL-Antikörpern durch erhebliche, teils schwere Nebenwirkungen belastet. Die vorliegende Erfindung stellt pharmazeutische Zusammensetzungen bereit, die die Behandlung mit Anti-RANKL-Antikörpern in puncto Wirksamkeit und Sicherheit erheblich verbessern. Die pharmazeutischen Zusammensetzungen umfassen neben Anti-RANKL-Antikörpern Kalzium und Vitamin D.

Anti-RANKL-Antikörper binden an RANKL. RANKL ist ein Transmembranprotein, kann jedoch ebenfalls als lösliches Protein auftreten. RANKL spielt eine entscheidende Rolle bei der Bildung, der Funktion und dem Überleben von Osteoklasten. Beim multpilem Myelom und bei Knochenmetastasen tritt eine erhöhte Osteoklastenaktivität auf, die durch RANKL stimuliert wird. Dies führt zu erhöhtem Knochenabbau.

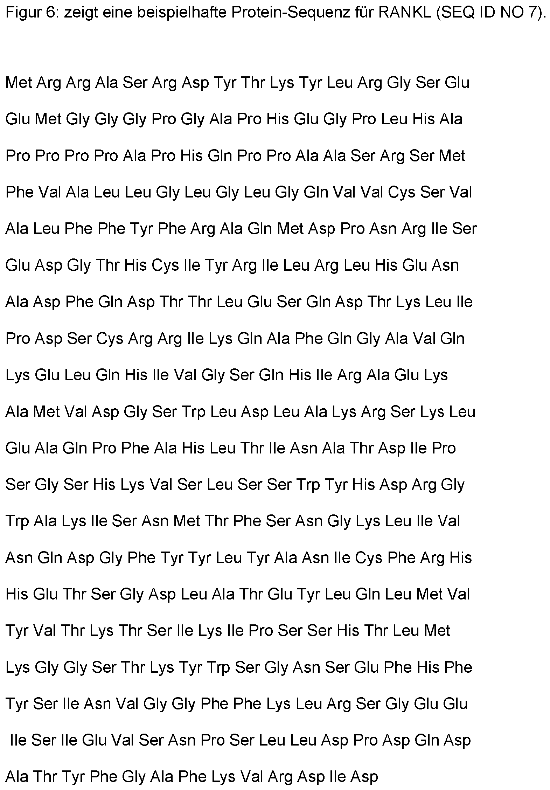

Die Sequenz des humanen RANKL-Proteins ist dargestellt in Figur 6. Das Protein liegt in verschiedenen Isoformen vor. Bei Isoform 2 fehlen die Aminosäuren 1-73 und bei Isoform 3 die Aminosäuren 1-47.

Anti-RANKL-Antikörper verhindern die RANK-RANKL-Interaktion. Dadurch wird die Anzahl und die Funktion der Osteoklasten reduziert und der Knochenabbau vermindert.

Der Begriff "Anti-RANKL-Antikörper" umfaßt alle RANKL-bindenden Moleküle, die an mindestens ein Epitop von RANKL binden und auf diese Weise die Aktivität von RANKL beeinflussen, d.h. die Bindung von RANKL an RANK blockieren oder vermindern, so dass eine Aktivierung der Osteoklasten vermindert oder verhindert wird. Eine ausgewählte humane Aminosäure-Sequenz von RANKL ist abgebildet in Figur 6 (SEQ ID NO 7).

Der Begriff "Epitop" bezeichnet eine Bindungsstelle auf einem Antigen, in diesem Fall RANKL, an die der Anti-RANKL-Antikörper spezifisch bindet. Das Epitop kann an eine primäre Aminosäuresequenz von RANKL binden, die typischerweise mindestens 3 Aminosäuren, üblicherweise mindestens 5 oder zum Beispiel 8-10 Aminosäuren umfaßt. Je nach der 3-dimensionalen Struktur von RANKL kann das Epitop auch Aminosäuren binden, die nicht in der Sequenz aufeinander folgen, sondern aufgrund der 3-dimensionalen Struktur in der Nähe befindlich sind.

Der Begriff "spezifisch bindet" bedeutet, dass der Antikörper in der Lage ist, mit mindestens 2, vorzugsweise 3, stärker bevorzugt 4 Aminosäuren eines Epitops zu interagieren, zum Beispiel nach dem "Schlüssel-Schloß-Prinzip".

Der Begriff "spezifisch" bedeutet, dass der Antikörper an ein RANKL-Molekül bindet und im Wesentlichen nicht an andere Proteine oder Moleküle. Dabei kann es vorkommen, dass der Antikörper kreuzreagiert mit RANKL-Molekülen von anderen Spezies. Der Antikörper soll jedoch nicht an andere Moleküle binden wie zum Beispiel RANK. Eine Bindung wird im Allgemeinen als spezifisch angesehen, wenn die Bindungsaffinität größer als 10⁻⁵ M beträgt. Bevorzugt ist die spezifische Bindungsaffinität ca. 10⁻¹¹ bis 10⁻⁸ M (KD), bevorzugt ca. 10⁻¹¹ bis 10⁻⁹ M.

Der Begriff "bindet im Wesentlichen nicht" bedeutet, dass der Anti-RANKL-Antikörper dieser Erfindung kein anderes Protein bindet, insbesondere eine Kreuzreaktivität von weniger als 30% aufweist, bevorzugt 20%, stärker bevorzugt 10%, insbesondere weniger als 9,8,7,6, oder 5% ein anderes Protein oder Molekül bindet.

Der Begriff "Polypeptid" ist geichbedeutend mit dem Begriff "Protein". Proteine enthalten ein oder mehrere Aminosäuren, die durch kovalente Bindungen miteinander verbunden sind. Sie können post-translationalen Modifikationen wie beispielsweise Glykosylierungen unterworfen sein. Dies ist allgemein bekannt.

Der Begriff "Antikörper" umfaßt ein Protein, das eine oder mehrere Antigen-bindende Domänen aufweist, mindestens teilweise durch Immunglobulingene oder Teile davon kodiert wird. Ein "Immunglobulin" ist ein "Antikörper". Der Antikörper umfaßt typischerweise ein glykosiliertes Tetramer (Proteine), das aus 2 leichten Ketten mit ungefähr je 25 kDa und 2 schweren Ketten mit ungefähr 50 kDA bestehen. 2 Typen von leichten Ketten existieren in Antikörpern: lambda und kappa. Die leichten Ketten bestehen aus jeweils einer variablen und einer konstanten Domäne. Bezeichnet werden diese als VL und CL. Abhängig von der Aminosäuresequenz der konstanten Region der schweren Ketten, werden Immunglobuline in 5 Klassen eingeteilt: A, D, E, G und M und einige werden in weitere Untergruppen aufgeteilt, beispielsweise IgG1, IgG2, IgG3, IgG4, IgA1 und IgA2. Die schweren Ketten haben jeweils eine variable und drei bei IgG und IgA bzw. vier konstante Domänen bei IgM und IgE. Bezeichnet werden diese analog als VH und CH1, CH2, CH3.

IgM-Antikörper enthalten 10 Antigen-Bindungsstellen. Die Grundeinheit besteht aus zwei schweren H-Ketten und zwei leichten L-Ketten. IgM wird als erstes Immunglobulin während einer Immunantwort produziert und aktiviert das Komplementsystem. Körpereigene, natürlich produzierte IgM-Antikörper werden von B1-Zellen sezerniert (CD20⁺, CD20⁺, CD27⁺, CD43⁺ und CD70⁺-Zellen). Neben der Abwehr eindringender Mikroorganismen, sind sie an der Gewebehomöostase durch Clearance apoptotischer und veränderter Zellen durch komplementabhängige Mechanismen beteiligt. Sie hemmen Entzündungsprozesse und entfernen veränderte Zellen.

IgA-Antikörper enthalten 2-5 der 4-Ketten-Einheiten, die Einheiten zusammen mit der J-Kette bilden können. IgA kann als Monomer (also nur ein Molekül) oder als Dimer (zwei Moleküle, verbunden an den langen Enden des Antikörper-Ypsilon) vorkommen. Bei den Dimeren handelt es sich um das sogenannte sekretorische IgA (slgA).

Jede leichte Kette enthält eine N-terminale variable (V)-Domäne (VL) und eine konstante Domäne (CL). Jede schwere Kette enthält eine N-terminale V-Domäne (VH), 3 oder 4 C-Domänen (CHs) und eine Hinge-Region.

Die konstanten Domänen des Antikörpers sind nicht direkt an der Antigenbindung beteiligt, können aber diverse Effektorfunktionen haben, beispielsweise bei der antikörperabhängigen zellulären Toxizität. Falls ein Antikörper die zelluläre Toxizität ausübt, handelt es sich bevorzugt um den IgG1-Subtyp, während der IgG4-Subtyp diese Fähigkeit nicht aufweist.

Die Verbindung von VH und VL bildet eine antigenbindende Stelle. Jede L-Kette ist über eine kovalente Disulphidbrücke an die H-Kette gebunden, während die beiden H-Ketten durch eine oder mehrere Disulphidbrücken miteinander verbunden sind, abhängig vom H-Ketten-Isotyp. Die VH und VL-Domänen bestehen aus vier Regionen mit relativ hoch konservierten Sequenzen (FR1, FR2, FR3 und FR4), die das Gerüst für die drei Regionen mit hypervariablen Sequenzen bilden, den complementary determining regions, CDRs. Die CDRs sind im Wesentlichen für die Interaktion zwischen Antigen und Antikörper verantwortlich. CDRs werden näher bezeichnet als CDR1, CDR2 und CDR3. Dementsprechend werden CDR-Regionen der schweren Kette als H1, H2 und H3 bezeichnet, während die CDR-Regionen der leichten Kette als L1, L2 und L3 bezeichnet werden.

Der Begriff "variabel" bezeichnet den Teil der Immunglobulin-Domäne, der Variabilität in der Sequenz enthält und die Spezifität und die Bindungsaffinität eines bestimmten Antikörpers definiert, wie beispielsweise variable Domänen. Die Variabilität ist im Antikörper nicht gleichmäßig verteilt, sie konzentriert sich auf die Sub-Domänen der jeweiligen leichten und schweren Ketten, welche als hypervariable Regionen bezeichnet werden oder CDRs.

Die weniger konservierten Regionen bzw. die nicht-hypervariablen Regionen der variablen Domänen sind die sogenannten Framework Regionen (FR), die häufig eine Beta-Faltblattstruktur aufweisen. Die FR-Regionen und die CDRs bilden die Struktur, die das Antigen erkennt und bindet (Kabat E.A., Wu,T.T., Perry,H., Gottesman,K. and Foeller,C. (1991) Sequences of Proteins of Immunological Interest, Fifth Edition. NIH Publication No. 91-3242). Die konstanten Domänen sind nicht direkt in die Antigen-Antikörperbindung involviert, vermitteln allerdings andere Reaktionen wie beispielsweise die antikörperabhängige und zellvermittelte Zytotoxizität und Komplementaktivierung.

Der Begriff "CDR" bzw. "CDRs" bezeichnet die complementary determining region (CDR). Die CDR werden, wie oben dargestellt, als CDR 1-3 bezeichnet, diejenigen der leichten Kette als CDRL1, CDRL2 und CDRL3 und die variablen Regionen der schweren Kette beinhalten als CDRH1, CDRH2 und CDRH3. CDRs tragen maßgeblich zur Aktivität des Antikörpers bei. Die exakte Länge der CDRs wird in den verschiedenen Systemen teils unterschiedlich klassifiziert und nummeriert. CDRs werden nachfolgend nach Kabat, Chothia bezeichnet. Alle Systeme haben Überlappungen, was die Bezeichnung der hypervariablen Region innerhalb der variablen Sequenz angeht (Kabat et al., Chothia et al., J. Mol. Biol., 1987, 196; 901 und Mac Callum et al., J. Mol. Biol., 1996, 262:732) vorliegend wird im Wesentlichen auf die Nummerierung von Kabat Bezug genommen.

Der Begriff "Aminosäure" oder "Aminosäurerest" bezeichnet Aminosäuren wie in der Fachwelt bekannt, ausgewählt aus der Gruppe Alanin (ala oder A); Arginin (Arg oder R); Asparagin (Asn oder N); Aspartat, (Asp oder D); Cystein (Cys oder C), Glutamin (Gln oder Q); Glutaminsäure (Glu oder E); Glyzin (Gly oder G); Histidin (His oder H); Isoleucin (Ile oder I); Leucin (Leu oder L); Lysin (Lys oder K); Methionin (Met oder M); Phenylalanin (Phe oder F); Prolin (Pro oder P); Serin (Ser oder S); Threonin (Thr oder T); Tryptophan (Trp oder W); Tyrosin (Tyr oder Y); Valin (Val oder V), wobei modifizierte, synthetische oder seltene Aminosäuren ebenfalls verwendet werden können.

Der Begriff "hypervariable Region" oder "CDR" bezeichnet die Abschnitte solcher Regionen bei den leichten (L-Ketten) und schweren Ketten (H-Ketten) der Immunglobuline, die große Unterschiede in der Aminosäuresequenz aufweisen. Es sind diejenigen Regionen eines Antikörpers oder T-Zell-Rezeptors, die für die Erkennung des Antigens zuständig sind. Die CDRs sind die variabelsten Teile der Rezeptoren und sind für deren Vielfalt verantwortlich. Die drei komplementaritätsbestimmenden Regionen CDR1, CDR2 und CDR3 sind Schleifen am Ende einer V-Domäne von Antikörpern. Sie treten mit einem Antigen in direkten Kontakt. In der Fachwelt sind mindestens zwei Methoden bekannt, CDRs zu identifizieren: ein Ansatz basiert auf Kreuz-Spezies Sequenzvariabilität (Kabat et al.) und ein anderer Ansatz basiert auf kristallographischen Studien des Antigen-Antikörper-Komplexes (Chothia, C. et al., J. Mol. Biol., 196:901-917 (1987)). Generell wird bevorzugt, die CDR-Identifizierung in Übereinstimmung mit der sogenannten Kabat-Nummerierung vorzunehmen.

Der Begriff "Framework-Region" ist der Fachperson bekannt und bezeichnet die Teile der variablen Region des Antikörpers die zwischen den hypervariablen Regionen, den CDRs besteht. Solche Framework-Regionen werden typischerweise als Framework-Regionen 1-4 bezeichnet (FR1, FR2, FR3 und FR4) und bilden das Gerüst für die 6 CDRs, drei der leichten Kette und drei der schweren Kette) im dreidimensionalen Raum, um eine antigen-bindende Oberfläche bereitzustellen.

Die Erfindung umfaßt sämtliche Anti-RANKL-Antikörper, die nach dem Kabat-Nummerierungssystem als austauschbar gelten und antigen-bindende Funktion haben, d.h. an ein RANKL-Epitop binden können.

"Antikörper" bedeutet ein monoklonaler, polyklonaler, monospezifischer, bispezifischer, bifunktionaler, einzelkettiger, synthetischer, rekombinanter, mutierter, humaner, humanisierter, chimärer Antikörper (Harlow and Lane, "Antibodies, A Laboratory Manual", CSH Press, Cold Spring Harbor, USA), der an ein RANKL-Molekül bindet oder ein Derivat, das die Bindungskapazität behält oder im Wesentlichen behält. Domain Antikörper (dAbs) und Nanobodies sind ebenfalls vom Begriff "Antikörper" umfaßt.

Ein bispezifischer oder bifunktionaler Antikörper ist ein künstlicher Hybrid-Antikörper mit 2 verschiedenen schweren/leichten Ketten und zwei verschiedenen Bindungsstellen. Der Begriff und die Methoden der Herstellung sind der Fachperson bekannt (z.B. Songsivilai & Lachmann, Clion. Exp. Immunol. 79:315-321 (1990); Kostelny et al., J. Immunol. 148, 1547-1553 (1992).

Die Herstellung monoklonaler Antikörper ist der Fachwelt bekannt (z.B. Kohler und Milstein (1975), Nature, 256:495-499), weitere Methoden beinhalten den sogenannten Phage Display (Ladner et al., US Patent No. 5,223,409).

Bevorzugte Derivate dieser Antikörper sind chimäre Antikörper, die zum Beispiel eine variable Region der Maus oder der Ratte und eine humane konstante Region umfassen. Der Begriff "Antikörper" umfasst ebenfalls einen bifunktionalen oder bispezifischen Antikörper und Antikörper-Konstrukte wie Fvs (scFv) aus Einzelketten oder Antikörper-Fusionsproteine. Der Begriff "scFv" (single chain Fv Fragment) ist der Fachperson bekannt und bevorzugt, da das Fragment rekombinant hergestellt werden kann.

Der Antikörper kann human oder humanisiert sein. Der Begriff "humanisierter Antikörper" bedeutet, dass mindestens eine Antikörperbindungsstelle (complementary determining region (CDR)) wie z.B. CDR3 und vorzugsweise alle 6 CDRs ausgetauscht wurden durch CDRs von einem humanen Antikörper mit der gewünschten Spezifität. Optional wurden die nicht-humane(n) konstante(n) Region(en) des Antikörpers ersetzt durch die konstante(n) Region(en) eines humanen Antikörpers. Methoden zur Herstellung von humanisierten Antikörpern sind beschrieben z.B. in EP 0239400 A1 und WO 90/07861.

Alternativ zum humanisierten Antikörper wird ein humaner Antikörper bereitgestellt. Transgene Tiere wie beispielsweise Mäuse sind in der Lage nach der Immunisierung humane Antikörper herzustellen ohne typische Maus-Antikörpersequenzen. Sie können vollständig humane Antikörper bilden (z.B. Jakobovits et al., Proc. Natl. Acad. Sci. USA, 90:2551 (1993)). Alternativ kann die sogenannte Phage-Display-Technologie verwendet werden, humane Antikörper oder antigen-bindende Fragmente zu produzieren. Die Techniken sind bekannt (Mc Cafferty et al., Nature 348:552-553 (1990); Johnson, Kevin S. and Chiswell, David J., Curr. Opin. Struct. Biol. 3:564-571 (1993)).

Der Begriff Antigen-bindendes Fragment bezeichnet ein Fragment des oben definierten Begriffs "Antikörper" wie z.B. getrennte leichte und schwere Ketten, Fab, Fab/c, Fv, scFv, Fd, dAb, Fab', F(ab')2. Ein antigen-bindendes Fragment kann eine variable Region der leichten Kette und eine variable Region der schweren Kette umfassen, nicht notwendigerweise beide zugleich.

Verfahren zur Antikörperproduktion und -isolation sind fachbekannt, siehe z.B. Harlow und Lane; Antibodies: A Laboratory Manual, Cold Spring Harbor Laboratory, New York (1988). Allgemeine Antikörperreinigungsverfahren umfassen unter anderem Salzpräzipitation (zum Beispiel mit Ammoniumsulfat); lonenaustauschchromatographie (zum Beispiel auf einer Kationen- oder Anionenaustauschsäule, die bei neutralem pH laufen gelassen wird und mit Schrittgradienten zunehmender lonenstärke eluiert wird); Gelfiltrationschromatographie (einschließlich Gelfiltrations-HPLC) und Chromatographie auf Affinitätsharzen wie z.B. Protein A, Protein G, Hydroxylapatit oder Anti-Ig. Antikörper können auf Affinitätssäulen, die einen Antigenabschnitt enthalten, ebenfalls gereinigt werden. Bevorzugte Fragmente werden unter Einsatz von Protein-A-CL-Sepharose-4B-Chromatographie gefolgt von Chromatographie auf einer DEAE-Sepharose-4B-lonenaustauschsäule gereinigt.

Die Erfindung umfasst auch Hybridantikörper, in welchen ein Paar von H- und L-Ketten aus einem ersten Antikörper erhalten wird, während das andere Paar von Hund L-Ketten aus einem anderen zweiten Antikörper erhalten wird. Für die Zwecke der Erfindung stammt ein Paar von L- und H-Ketten aus Anti-RANKL-Antikörpern. In einem Beispiel bindet jedes L-H-Kettenpaar verschiedene Epitope eines RANKL-spezifischen Antigens. Solche Hybride können auch unter Einsatz humanisierter H- oder L-Ketten gebildet werden. Die Erfindung umfasst auch andere bispezifische Antikörper, wie z.B. jene, die zwei separate Antikörper enthalten, die durch ihre konstanten Regionen kovalent gebunden sind.

Die Größe des antigenbindenden Fragments kann nur die Minimalgröße sein, die erforderlich ist, um eine gewünschte Funktion bereitzustellen. Sie kann gegebenenfalls eine weitere Aminosäuresequenz umfassen, entweder nativ zum antigenbindenden Fragment oder aus einer heterologen Quelle, wie gewünscht. Anti-RANKL-antigenbindende Fragmente können 5 aufeinander folgende Aminosäurereste aus einer Antikörper-V-Regionsequenz umfassen. Es sind ebenfalls Polypeptide umfasst, die 7 Aminosäurereste, noch bevorzugter etwa 10 Aminosäurereste, noch bevorzugter etwa 15 Aminosäurereste, noch bevorzugter etwa 25 Aminosäurereste, noch bevorzugter etwa 50 Aminosäurereste, noch bevorzugter etwa 75 Aminosäurereste aus der Antikörper-L- oder H-Ketten-V-Region umfassen. Noch bevorzugter sind Polypeptide, welche die gesamte Antikörper-L- oder H-Ketten-V-Region umfassen.

Substitutionen können von der Veränderung oder Modifikation eines oder mehrerer Aminosäurereste bis zum vollständigen Neuentwurf einer Region wie z.B. der V-Region reichen. Aminosäurerestsubstitutionen, wenn sie vorhanden sind, sind vorzugsweise konservative Substitutionen, welche die Faltung oder funktionelle Eigenschaften des Peptids nicht negativ beeinflussen. Gruppen von funktionell verwandten Aminosäureresten, innerhalb welcher konservative Substitutionen gemacht werden können, sind Glycin/Alanin, Valin/Isoleucin/Leucin; Asparagin/Glutamin, Asparaginsäure/Glutaminsäure; Serin/Threonin/Methionin; Lysin/Arginin und Phenylalanin/Tyrosin/Tryptophan. Antikörper können glykosyliert oder unglykosyliert sein, nach Translation modifiziert werden (z.B. Acetylierung und Phosphorylierung) oder synthetisch modifiziert werden (z.B. die Bindung einer Markierungsgruppe).

Es werden ferner Anti-RANKL-Antikörper bereitgestellt, die Aminosäuresequenzänderungen enthalten. Diese Änderungen können Deletionen, Insertionen, Substitutionen einzelner oder mehrerer Aminosäuren umfassen. Dadurch können post-translationale Modifikationen ebenfalls Änderungen unterworfen sein.

Änderungen in den CDRs der leichten und schweren Kette sind am sinnvollsten, insbesondere in den hypervariablen Regionen, FR-Änderungen in der leichten und/oder schweren Kette können jedoch ebenfalls in Betracht gezogen werden. Umfasst eine CDR-Sequenz 6 Aminosäuren, können 1-3 Aminosäuren geändert werden. Wenn sie 15 Aminosäuren enthält können 1-6 Aminosäuren geändert werden. Wenn CDRs in der leichten und/oder schweren Kette geändert werden, soll die geänderte Aminosäuresequenz mindestens 60%, stärker bevorzugt 65%, noch stärker bevorzugt 70%, besonders bevorzugt 75%, am stärksten bevorzugt 80% identisch mit der ursprünglichen CDR-Sequenz sein. Daher hängt es von der Länge des jeweiligen CDRs ab, wie hoch die Identität mit dem ursprünglichen CDR ist. Während ein CDR mit 5 Aminosäuren und einer Änderung zu 80% identisch ist, ist die Änderung bei einem längeren CDR prozentual niedriger bei einer Änderung von einer Aminosäure. CDRs können demzufolge unterschiedlich hohe Anteile an Identität zum ursprünglichen CDR haben, z.B. CDRH1 kann zu 90% identisch sein, während CDRL2 zu 83% identisch ist.

Bevorzugte Änderungen des Anti-RANKL-Antikörpers können Tabelle 1 entnommen werden. Tabelle 1:

| Originalaminosäure | Beispielhafte Substitutionen | Bevorzugte Subsitutionen |
|---|---|---|
| Ala | Val;Leu;lle | Val |
| Arg | Lys;Gln;Asn | Lys |
| Asn | Gln;His;Asp;Lys;Arg | Gln |
| Asp | Glu;Asn | Glu |
| Cys | Ser;Ala | Ser |
| Gln | Asn;Glu | Asn |
| Glu | Asp;Gln | Asp |
| Gly | Ala | Ala |
| His | Asn;Gln;Lys;Arg | Arg |
| Ile | Leu;Val;Met;Ala;Phe | Leu |
| Leu | Norleucin;Ile;Val;Met;Ala | Ile |
| Lys | Arg;Gln;Asn | Arg |
| Met | Leu;Phe;Ile | Leu |
| Phe | Leu;Val;Ile;Ala;Tyr | Tyr |
| Pro | Ala | Ala |
| Ser | Thr | Thr |
| Thr | Ser | Ser |
| Trp | Tyr;Phe | Tyr |
| Tyr | Trp;Phe;Thr;Ser | Phe |
| Val | Ile;Leu;Met;Phe;Ala | Leu |

Diese sind beispielhaft aufgelistet und sind vorzugsweise zu 60%, stärker bevorzugt zu 65%, noch stärker bevorzugt zu 70%, noch stärker bevorzugt zu 75% und am stärksten bevorzugt zu 80% identisch zur ursprünglichen CDR-Sequenz. Die Sequenzen sind umfaßt, die an RANKL spezifisch binden. Die Methoden über Änderungen in den CDRs sind im Übrigen bekannt (z.B. US Patent 6,180,370; 5,693,762;5,693,761; 5,585,089 und 5,530,101).

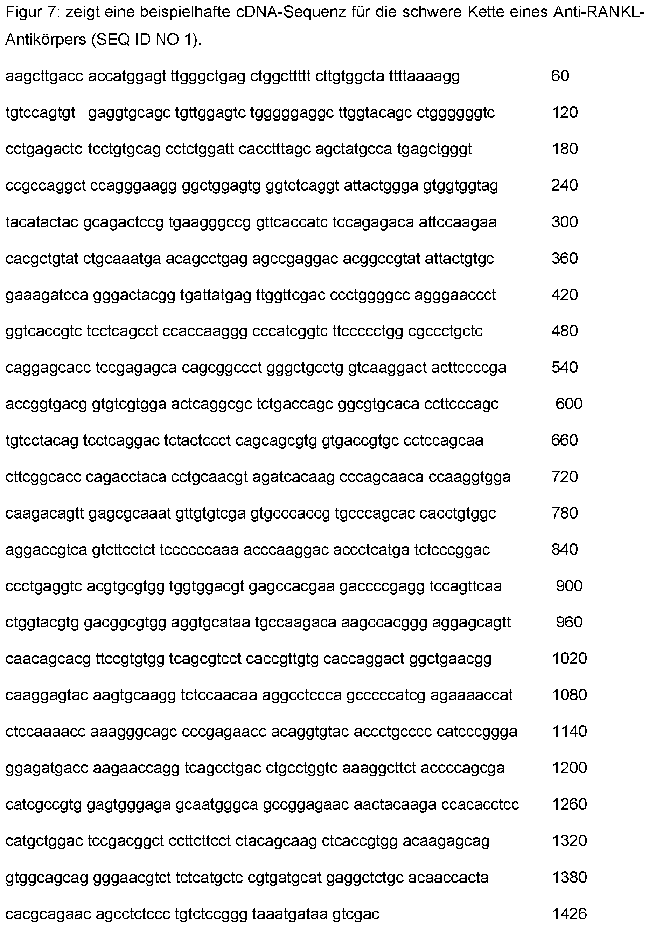

Eine beispielhafte cDNA-Sequenz für die schwere Kette eines Anti-RANKL-Antikörpers ist dargestellt in Figur 7 (SEQ ID NO 1).

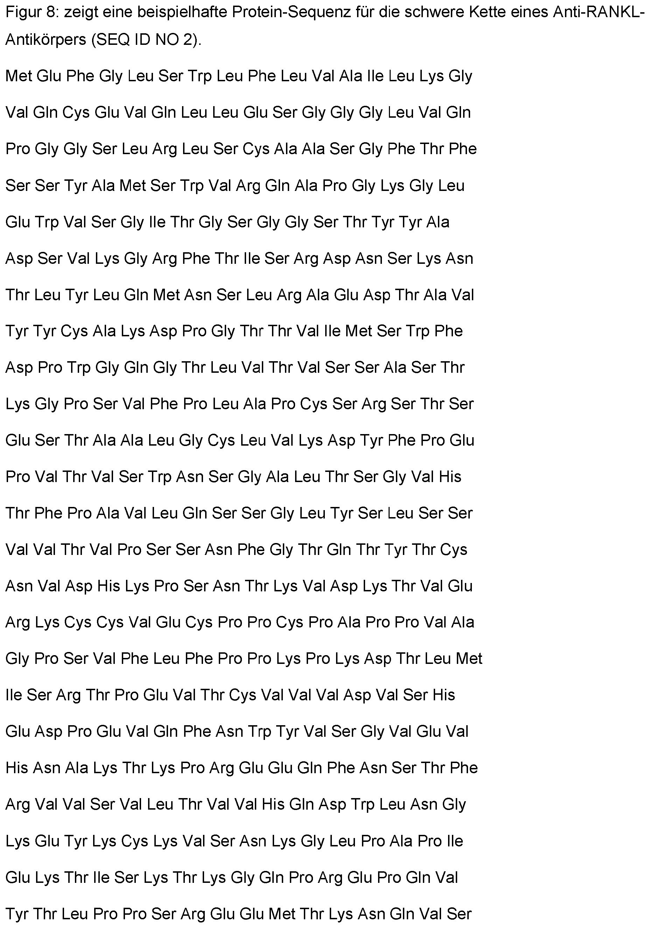

Eine beispielhafte Protein-Sequenz für die schwere Kette eines Anti-RANKL-Antikörpers ist dargestellt in Figur 8 (SEQ ID NO 2).

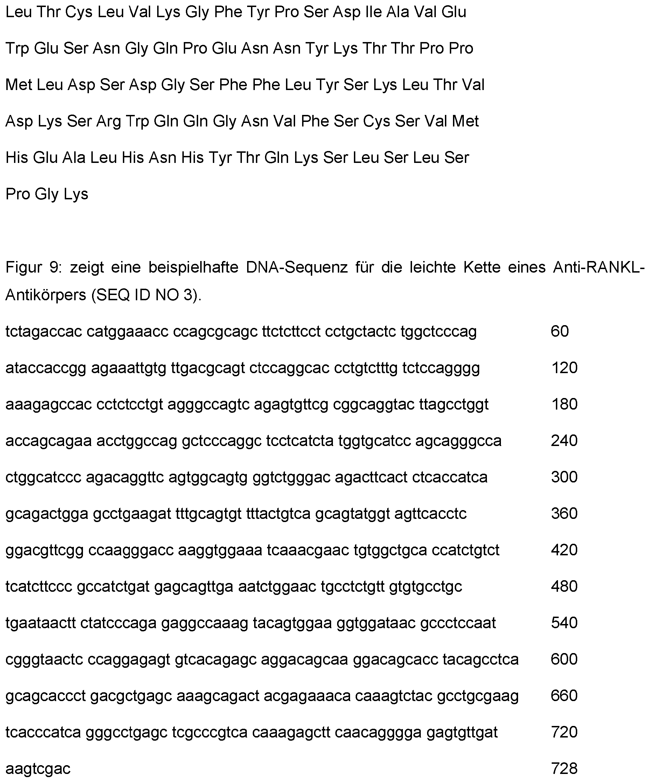

Eine beispielhafte DNA-Sequenz für die leichte Kette eines Anti-RANKL-Antikörpers ist dargestellt in Figur 9 (SEQ ID NO 3).

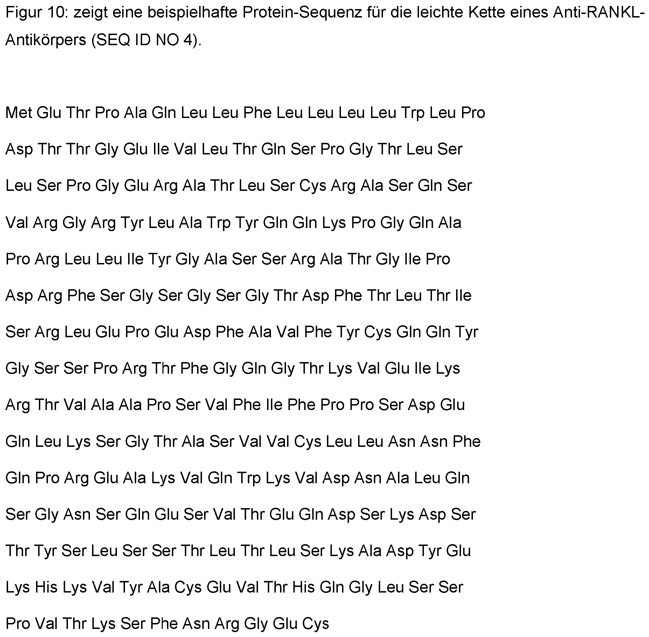

Eine beispielhafte Protein-Sequenz für die leichte Kette eines Anti-RANKL-Antikörpers ist dargestellt in Figur 10 (SEQ ID NO 4).

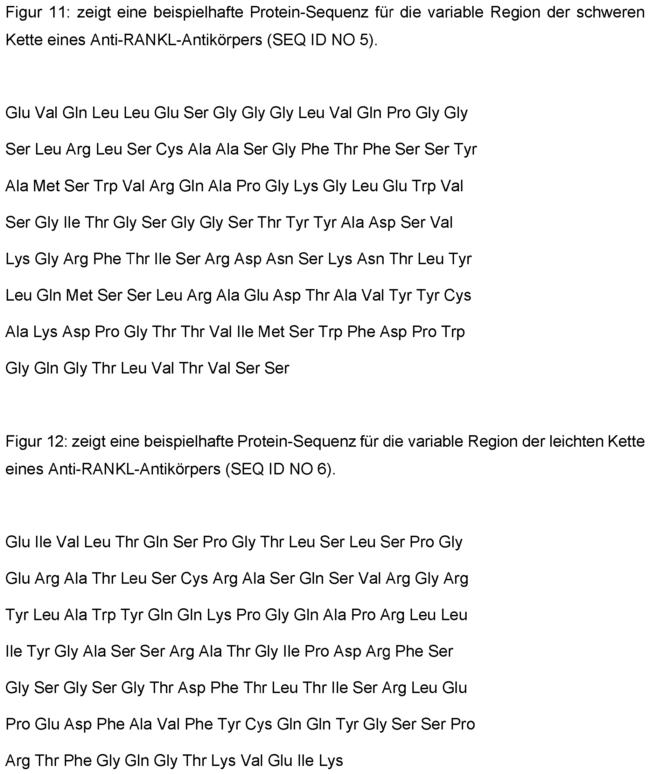

Eine beispielhafte Protein-Sequenz für die variable Region der schweren Kette eines Anti-RANKL-Antikörpers ist dargestellt in Figur 11 (SEQ ID NO 5).

Eine beispielhafte Protein-Sequenz für die variable Region der leichten Kette eines Anti-RANKL-Antikörpers ist dargestellt in Figur 12 (SEQ ID NO 6).

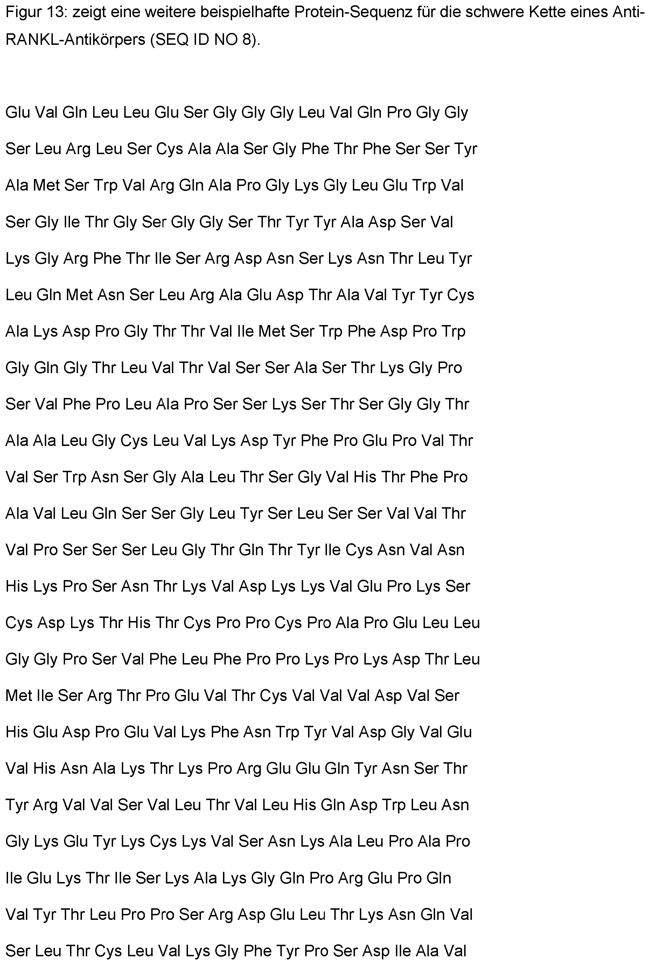

Eine beispielhafte Protein-Sequenz für RANKL ist dargestellt in Figur 13 (SEQ ID NO 7).

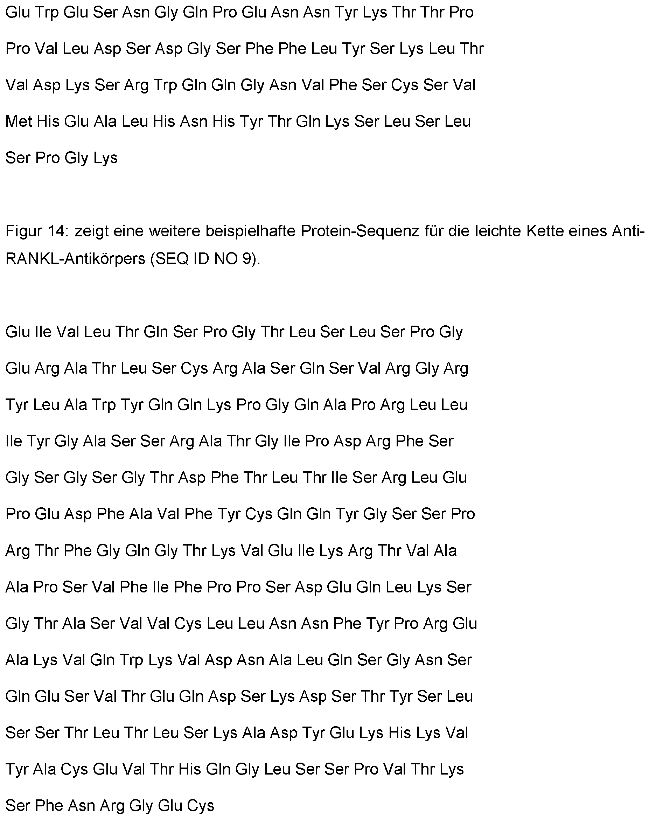

Eine weitere beispielhafte Protein-Sequenz für die schwere Kette eines Anti-RANKL-Antikörpers ist dargestellt in Figur 14 (SEQ ID NO 8).

Eine weitere beispielhafte Protein-Sequenz für die leichte Kette eines Anti-RANKL-Antikörpers ist dargestellt in Figur 15 (SEQ ID NO 9).

Erfindungsgemäß sind ebenfalls die antigenbindenenden Fragmente des Anti-RANKL-Antikörpers umfasst.

Prolia (enthält Denosumab, Anti-RANKL-Antikörper und ist als Arzneimittel zugelassen) wird von der Amgen GmbH in Verkehr gebracht. Es enthält 60mg Denosumab Injektionslösung. Jede Fertigspritze enthält 60 mg Denosumab in 1 ml Lösung (60 mg/ml). Die empfohlene Dosis von Prolia ist 60 mg, angewendet als einzelne subkutane Injektion einmal alle 6 Monate (Oberschenkel, Abdomen oder Oberarm). Prolia wird zur Behandlung der Osteoporose bei postmenopausalen Frauen zur Verhinderung vertebraler und nichtvertebraler Frakturen, zur Behandlung zur Erhöhung der Knochenmineraldichte bei Männern mit Osteoporose und erhöhtem Frakturrisiko angewendet, sowie als Begleitbehandlung bei Frauen mit Brustkrebs unter adjuvanter Behandlung mit Aromatasehemmern und bei Männern mit Prostatakarzinom unter Hormonablationstherapie, wenn ein erhöhtes Frakturrisiko vorliegt.

Xgeva, Arzneimittel, enthält ebenfalls Anti-RANKL-Antikörper (Denosumab, Amgen, jede Durchstechflasche enthält 120mg Denosumab in 1,7ml Lösung (70mg/ml)). Xgeva wird zur Prävention skelettbezogener Komplikationen (pathologische Fraktur, Bestrahlung des Knochens, Rückenmarkskompression oder operative Eingriff am Knochen) bei Erwachsenen mit Knochenmetastasen aufgrund solider Tumoren angewendet und bei der Behandlung von Erwachsenen und skelettal ausgereiften Jugendlichen mit Riesenzelltumoren des Knochens, die nicht resezierbar sind oder bei denen eine operative Resektion wahrscheinlich zu einer schweren Morbidität führtln der Fachinformation wird lediglich eine ausreichende Einnahme von Kalzium und Vitamin D empfohlen, die genaue Dosierung wird nicht angegeben.

Die ausreichende tägliche Gesamtzufuhr an Kalzium beträgt 1000 mg. Über die Nahrung wird Kalzium aufgenommen, sodass bei kalziumreicher Ernährung die erforderliche Tagesdosis erreicht werden kann. Eine Kalziumzufuhr von mehr als 2500mg pro Tag wird als problematisch angesehen, weil sich dadurch unter anderem das Risiko für die Bildung von Nierensteinen erhöhen kann. Aufgrund diverser Veröffentlichungen über kardiovaskuläre Nebenwirkungen durch die übermäßige Einnahme von Kalzium, haben viele Patienten davon Abstand genommen, Kalzium in ausreichender Menge, selbst unter Behandlung mit Anti-RANKL-Antikörpern, zu sich zu nehmen.

Vielfach wird dadurch der Behandlungserfolg von Anti-RANKL-Antikörpern insgesamt gefährdet und die kardiovaskulären Nebenwirkungen nun durch die Unterversorgung mit Kalzium hervorgerufen bzw. durch die starke Hemmung des Knochenabbaus durch die Behandlung mit Anti-RANKL-Antikörpern, die eine Resorption des Kalziums aus dem Knochen ins Blut hemmt.

Dies führt zu Hypokalzämien, die kardiovaskuläre Nebenwirkungen hervorrufen. Im Prinzip wird damit genau das Gegenteil des erwünschten Erfolgs eintreten. Durch den Kalziummangel -nicht durch die Überversorgung mit Kalzium- treten nun vermehrt kardiovaskuläre Nebenwirkungen auf, die durch eine angemessene Versorgung mit Kalzium bei der Behandlung mit Anti-RANKL-Antikörpern hätten verhindert werden können.

Eine Supplementation mit einer pharmazeutischen Zusammensetzung mit 1000-1500mg Kalzium pro Tag wird bei ausreichend kalziumhaltiger Ernährung zu einer Überversorgung mit Kalzium führen, die das Risiko der kardiovaskulären Nebenwirkungen erhöht. Entgegen der Studiendesigns, die in der Fachinformation von Aclasta eine Tagesdosis von 1000-1500mg Kalzium beinhalten, wird in der vorliegenden Erfindung eine niedrigere Dosierung bereitgestellt, um Hypokalzämien und kardiovaskuläre Nebenwirkungen zu behandeln bzw. diesen vorzubeugen.

Die bevorzugte Menge an Kalzium in der pharmazeutischen Zusammensetzung beträgt 400-600mg Kalzium pro Tag, besonders bevorzugt 500mg Kalzium pro Tag, um einerseits auch bei kalziumarmer Ernährung einen Grundspiegel an Kalzium zu erhalten und andererseits nicht in die Überversorgung zu geraten, die mit den genannten Risiken verbunden und demzufolge für die Patienten nicht akzeptabel ist. Dem Ergebnisbericht der in Deutschland durchgeführten "Nationale-Verzehrs-Studie II" zur Folge erreichen 46% der Männer und 55% der Frauen nicht die empfohlene tägliche Zufuhr von Kalzium. In der Altersgruppe zwischen 51-80 Jahre liegt die durchschnittliche Gesamtzufuhr in dem Bereich zwischen 490-1790 mg Kalzium pro Tag.

500mg Kalzium pro Tag sollen laut Fachinformation von Xgeva eingenommen werden, allerdings in der Kombination mit 400 I.E. Vitamin D pro Tag. Die Studien für Xgeva waren auf US-Patienten ausgerichtet. In den USA wird eine große Anzahl an Lebensmitteln mit Vitamin D supplementiert, so beispielsweise Milch, Orangensaft, Brotsorten oder Frühstücksflocken. In Deutschland und der EU ist dies im Wesentlichen nicht der Fall, so dass erfindungsgemäß eine höhere Menge an Vitamin D von 800-1200 I.E. pro Tag bereitgestellt wird, besonders bevorzugt 1000 I.E. Vitamin D pro Tag. Die höhere Menge an Vitamin D ist in der Lage, den Transport einer potentiell geringeren Menge an Kalzium aus dem Gastrointestinaltrakt ins Blut zu fördern und trotz der geringeren Menge an Kalzium zu einem ausreichenden Kalziumserumspiegel zu gelangen.

Entgegen der Fachinformation von Xgeva wird daher eine Kombinationstherapie aus Anti-RANKL-Antikörpern, 400-600mg Kalzium und 800-1200 I.E. Vitamin D täglich zur Prophylaxe und Behandlung der Hypokalzämie bereitgestellt. Das erhöhte Vitamin D stellt auf diese Weise sicher, dass die geringe Menge an Kalzium von 400-600mg, bevorzugt 500mg Kalzium täglich, den Kalziumspiegel im Normwert über 2,2 mmol/l (9 mg/dl) zu halten.

Der Vitamin D-Spiegel ist gut messbar. Die erfindungsgemäße Kombination wird für eine Vitamin D Serum-Konzentration bereitgestellt, die nicht unter 20ng/ml (50nmol/l Serum) liegt.

Bei einem schweren Mangel (<10ng/ml Serum) sollten 200.000 I.E. über 10 Tage und dann 20.000 I.E. wöchentlich substituiert werden. Bei einem deutlichen Mangel (10-20ng/ml Serum) sollte eine initiale Substitution von 100.000 I.E. erfolgen und dann eine Erhaltung von 20.000 I.E./Woche. Liegt ein Mangel (21-30ng/ml Serum) vor, sollte eine Substitution von 20.000 I.E./Woche erfolgen. Ist der normale Vitamin D-Spiegeln (31-60ng/ml Serum) erreicht, ist die tägliche erfindungsgemäße Substitution von 800-1200 I.E. Vitamin D, besonders bevorzugt 1000 I.E. anwendbar.

Eine Supplementation mit 400 I.E. Vitamin D ist nicht ausreichend, um den Vitamin D-Spiegel im normalen Bereich von 31-60ng/ml Serum bei Verabreichung der erfindungsgemäßen pharmazeutischen Zusammensetzung von Anti-RANKL-Antikörpern und Kalzium zu halten.

Auf diese Weise werden durch die erfindungsgemäße pharmazeutische Zusammensetzung aus Anti-RANKL-Antikörpern, Kalzium und Vitamin D Hypokalzämien verhindert.

Erfindungsgemäß wird die pharmazeutische Zusammensetzung umfassend Anti-RANKL-Antikörper zur Behandlung von Osteoporose, postmenopausaler Osteoporose, manifester Osteoporose, corticoinduzierter Osteoporose, Osteoporose des Mannes oder der Frau, Morbus Paget, Osteogenesis imperfecta, zur Prophylaxe von Frakturen der vorgenannten Erkrankungen, sowie zur Verwendung in der Behandlung und/oder Prophylaxe der durch Anti-RANKL-Antikörper therapiebedingten Hypokalzämie, verabreicht, vorzugsweise in einer Dosierung von 30 bis 90 mg in 1-2 ml Lösung (20-60 mg/ml), stärker bevorzugt 50-70 mg, noch stärker bevorzugt 60mg, bevorzugt in 1 ml Lösung (60 mg/ml) subkutan jährlich oder halbjährlich verabreicht.

Erfindungsgemäß wird die pharmazeutische Zusammensetzung umfassend Anti-RANKL-Antikörper im Bereich Onkologie zur Behandlung und/oder Prophylaxe skelettbezogener Komplikationen, insbesondere aufgrund von soliden Tumoren, vorzugsweise Mamma-, Prostata-, Lungen-, Darm- oder Knochenkarzinom (Osteosarkom), pathologischen Frakturen, Bestrahlung des Knochens, Rückenmarkskompression oder operativen Eingriffen am Knochen, Knochenmetastasen, Schmerzen bei Knochenmetastasen, Nerveneinklemmungen, Deformierungen aufgrund eines oder mehrerer solider Tumore wie beispielsweise Brustkrebs, Prostatakrebs, Lungenkrebs oder multiplem Myelom, sowie der Prophylaxe von Frakturen bei den vorgenannten Erkrankungen und zur Verwendung in der Behandlung und/oder Prophylaxe der durch Anti-RANKL-Antikörper therapiebedingten Hypokalzämie verabreicht, vorzugsweise in einer Dosierung von 60-180 mg in 1-3 ml Lösung, stärker bevorzugt 80-150 mg, noch stärker bevorzugt 120 mg, insbesondere in 1,7 ml Lösung (70 mg/ml) Anti-RANKL-Antikörper subkutan zur mindestens 3-4 wöchigen Anwendung verabreicht.

Erdfindungsgemäß werden die oben aufgeführten pharmazeutischen Zusammensetzungen verwendet, wobei der Anti-RANKL-Antikörper einen monoklonalen, polyklonalen, monospezifischen, bispezifischen, bifunktionalen, einzelkettigen, synthetischen, rekombinanten, mutierten, humanen, humanisierten, chimären, IgG, IgA, IgM oder IgE Antikörper, ein antigenbindendes Fragement oder ein Antikörper-Konstrukt wie Fvs (scFv) aus Einzelketten oder Antikörper-Fusionsproteinen bzw. ein Fragment davon, insbesondere getrennte leichte und schwere Ketten, Fab, Fab/c, Fv, scFv, Fd, dAb, Fab' oder F(ab')2 umfasst, wobei ein Fragment eine variable Region der leichten Kette und/oder eine variable Region der schweren Kette umfasst.

Der Begriff "Kalzium" bzw. "Calcium" umfasst alle bekannten pharmazeutisch verträglichen Kalzium-Verbindungen, insbesondere ohne darauf beschränkt zu sein Kalziumzitrat, weitere Bezeichnungen: Tricalciumcitrat, TCC, Tricalciumdicitrat, Tricalcium-di-[2-hydroxy-1,2,3-propantricarboxylat]-Tetrahydrat, E 333, C₁₂H₁₀Ca₃O₁₄, CAS-Nummern: 813-94-5 (wasserfrei), 5785-44-4 (Tetrahydrat); Calciumgluconat-Monohydrat (C₁₂H₂₂CaO₁₄ • H₂O, CAS-Nummern: 299-28-5 (wasserfrei) und 66905-23-5 (Monohydrat)); Calciumlactatgluconat, ein Doppelsalz der Milch- und Gluconsäure, das als Gemisch vorliegt, weitere Bezeichnungen: CLG, Calciumlaktatglukonat, Calciumlaktoglukonat, E 327, E 578, CAS-Nummern: 11116-97-5, 814-80-2 (Calciumlactat-Pentahydrat), 18016-24-5 (Calciumgluconat-Monohydrat), oder Kalziumkarbonat CaCO₃, CAS-Nummer: 471-34-1, Kalzium-Phosphat, Kalzium-Dihydrogenphosphat, Kalzium-Hydrogenphosphat, Kalzium-Hydrogenphosphat Dihydrat, Kalzium-Acetat, Kalzium-Ascorbat, Kalzium-Chloride, Kalzium-Glucoheptonat, Kalzium-Glycerophosphat und/oder Kalzium-Sulfat.

Der Begriff "Vitamin D" umfasst Vitamin D oder dessen Derivate, insbesondere, ohne darauf beschränkt zu sein, Vitamin D3 (Cholecalciferol, C₂₇H₄₄O, CAS-Nummer: 67-97-0), Calcitriol (1,25-Dihydroxyvitamin D3, 1α,25-Dihydroxycholecalciferol, 1,25(OH)₂Vitamin D3, 1,25(OH)₂D3, (5Z,7E)-(1S,3R)-9,10-Seco cholesta-5,7,10(19)-trien-1,3,25-triol, CAS-Nummer: 32222-06-3) oder 1α,25-Dihydroxycholecalciferol (biologisch aktive Form von Vitamin D3), Alphacalcidol (1α-Hydroxyvitamin D3), 24,25-Dihydroxyvitamin D3 oder Calcifediol (25-Hydroxyvitamin D3 25-Hydroxycholecalciferol, CAS-Nummer: 19356-17-3, IUPAC-Name: (6R)-6-[(1R,3aR,4E,7aR)-4-[(2Z)-2-[(5S)-5-Hydroxy-2-methylidene-cyclohexylidene] ethylidene]-7a-methyl-2,3,3a,5,6,7-hexahydro-1H-inden-1-yl]-2-methylheptan-2-ol), Vitamin D2 (3β,5Z,7E,22E)-9,10-Secoergosta-5,7,10(19),22-tetraen-3-ol, Calciferol, Ergo-Calciferol, CAS-Nummer: 50-14-6 bzw. dessen biologisch aktive Formen. Sowohl Vitamin D-Präparate, als auch Anti-RANKL-Antikörper (Denosumab, Prolia und Xgeva, Amgen Inc.) und Kalzium sind käuflich erwerblich und die Verfahren zur Herstellung derselben der Fachperson bekannt.

In einem weiteren Ausführungsbeispiel sind die pharmazeutischen Zusammensetzungen geeignet, zur Behandlung und/oder Prophylaxe von Nebenwirkungen der Anti-RANKL-Antikörper, insbesondere Kieferosteonekrosen einhergehend mit Schmerzen im Mund, nicht heilenden Wunden bis zur Auflösung des Kiefers, Hypokalzämien, kardiovaskulären Nebenwirkungen wie Herzrhythmusstörungen, Krämpfe, und sekundärer Hyperparathyreodismus, verwendet zu werden.

In einem Ausführungsbeispiel werden die oben angegebenen Mengen Anti-RANKL-Antikörper in einer Infusionslösung von 1-3ml in einer pharmazeutisch akzeptablen Lösung wie beispielsweise isotonischer Kochsalzlösung oder isotonischer Sorbitol-Natriumacetat-Polysorbat-Lösung, isotonischer Sorbitol-Natriumacetat-Lösung, isotonischer Glucoselösung oder anderen pharmazeutisch geeigneten isotonischen Lösungen geeigneten pH-Werts bereitgestellt.

In einem weiteren Ausführungsbeispiel werden Kalzium und Vitamin D mit pharmazeutisch geeigneten Hilfsstoffen bereitgestellt, insbesondere Lactose, Stärke, Säuerungsmitteln insbesondere Zitronensäure und Apfelsäure, Säureregulatoren insbesondere Natriumhydrogencarbonat und Natriumcarbonat, Feuchthaltemitteln insbesondere Sorbitol, Xylitol und Inulin, Trennmitteln insbesondere Tricalciumphosphat, Speisefettsäuren insbesondere Magnesiumsalze insbesondere Magnesiumstearat, natürliche und naturidentische und andere Aromen und Aromastoffe, Süßstoffe insbesondere Natrium-Cyclamat, Aspartam und Saccharin-Natrium, Maltodextrin, Farbstoffe insbesondere Rote-Bete-Saftpulver und Riboflavin-5'-phosphat, Siliciumdioxid insbesondere hochdispers, Siliciumdioxid hydrat, Phenylalanin, Arabisches Gummi, Saccharose, Gelatine, Maisstärke, Sojaöl, Glycerol, DL-alpha-Tocopherol, Isomalt, Natriumhydrogencarbonat, Natriumdihydrogencarbonat, Natriumcitrat, Natriumdihydrogencitrat, Carmellose Natrium, Acesulfam Kalium, Natriumascorbat, Triglyceride insbesondere mittelkettige, und/oder in pharmazeutisch verträglichen Flüssigkeiten, insbesondere Wasser, isotonischer Kochsalz-oder Glucoselösung bereitgestellt.

In einem weiteren Ausführungsbeispiel werden Anti-RANKL-Antikörper, Kalzium und Vitamin D, gegebenenfalls mit pharmazeutisch geeigneten Hilfsstoffen und/oder Flüssigkeiten, insbesondere als Brausetabletten, Schlucktabletten oder -kapseln, Kautabletten, Brausegranulaten, Direktgranulaten, Trinklösungen, Tropfen, Sublingualsprays, als Infusionslösungskonzentrate, Fertiginfusionen, Injektionslösungskonzentraten, Injektionslösungen oder Fertigspritzen einzeln oder zusammen bereitgestellt.

Die nachfolgenden Figuren und Beispiele dienen der Erläuterung ohne jedoch die Erfindung auf die Figuren oder Beispiele einzuschränken.

### Figuren:

Figur 1: zeigt eine Darstellung über die Kalziumzufuhr vom Gastrointestinaltrakt ins Blut und in den Knochen, sowie die beeinflussenden Faktoren, die eine Kalziumaufnahme fördern bzw. verringern.
Figur 2: zeigt die Folgen einer insuffizienten Kalziumaufnahme durch die Nahrung.
Figur 3: zeigt die Zu- und Abnahme der Knochenmasse im Verlauf des menschlichen Lebens und das erhöhte Knochenbruchrisiko im Alter mit Abnahme der Knochenmasse.
Figur 4: zeigt den erhöhten Kalziumbedarf mit zunehmendem Alter durch verringerte reale Kalziumaufnahme.
Figur 5: Eine Hypokalzämie liegt vor, wenn das Gesamtkalzium im Blutserum unter 2,2 mmol/l (9 mg/dl) liegt. Durch die medikamentöse Hemmung der Osteoklasten kann die Kalziumkonzentration im Blut nur noch über eine Zufuhr von Kalzium von außen (oral oder intravenös) ausgeglichen werden. Dies ist in Figur 5 dargestellt.
Figur 6: zeigt eine beispielhafte Protein-Sequenz für RANKL (SEQ ID NO 7).
Figur 7: zeigt eine beispielhafte cDNA-Sequenz für die schwere Kette eines Anti-RANKL-Antikörpers (SEQ ID NO 1).
Figur 8: zeigt eine beispielhafte Protein-Sequenz für die schwere Kette eines Anti-RANKL-Antikörpers (SEQ ID NO 2).
Figur 9: zeigt eine beispielhafte DNA-Sequenz für die leichte Kette eines Anti-RANKL-Antikörpers (SEQ ID NO 3).
Figur 10: zeigt eine beispielhafte Protein-Sequenz für die leichte Kette eines Anti-RANKL-Antikörpers (SEQ ID NO 4).
Figur 11: zeigt eine beispielhafte Protein-Sequenz für die variable Region der schweren Kette eines Anti-RANKL-Antikörpers (SEQ ID NO 5).
Figur 12: zeigt eine beispielhafte Protein-Sequenz für die variable Region der leichten Kette eines Anti-RANKL-Antikörpers (SEQ ID NO 6).
Figur 13: zeigt eine weitere beispielhafte Protein-Sequenz für die schwere Kette eines Anti-RANKL-Antikörpers (SEQ ID NO 8).
Figur 14: zeigt eine weitere beispielhafte Protein-Sequenz für die leichte Kette eines Anti-RANKL-Antikörpers (SEQ ID NO 9).

### Beispiel 1:

Ein Vitamin D-Mangel ist eine häufige Diagnose in der osteologischen Praxis. Zu Beginn der Therpaie wiesen 89 von 423 Patienten einen Vitamin D-Mangel auf, der sich unter dem Normwert von 20ng/ml Serum bzw. 50 nmol/l befand. Die Erhaltungsdosis betrug 1000 I.E. täglich Vitamin D. Eine Erhaltungsdosis von 400 I.E. Vitamin D täglich war nicht ausreichend, den Vitamin D-Spiegel auf dem Normwert zu halten. Tabelle 1 zeigt, dass eine Erhaltungsdosis von 1000 I.E. Vitamin D geeignet ist, den Vitamin D-Spiegel im Normbereich zu halten:

**Tabelle 1:**

| Patient | 25(OH) Vitamin D3-SerumspiegelAnfangswert | 25(OH) Vitamin D3-Serumspiegel bei Erhaltungsdosis 1000 I.E. Vitamin D täglich |
|---|---|---|
| 85 Jahre, weiblich | 9,4ng/ml | 25,5 ng/ml |
| 65 Jahre, weilblich | 8,8ng/ml | 23,4 ng/ml |
| 77 Jahre, weiblich | 12,4 ng/ml | 29,2 ng/ml |
| 81 Jahre, weiblich | 10,4ng/ml | 30,4 ng/ml |
| 75 Jahre, weiblich | 14,6ng/ml | 28,8 ng/ml |
| 86 Jahre, weiblich | 10,9ng/ml | 23,7ng/ml |
| 81 Jahre, weiblich | 8,6 ng/ml | 23,2ng/ml |
| 71 Jahre, weiblich | 17,1 ng/ml | 30,5 ng/ml |

### Beispiel 2:

Der Vitamin D-Spiegel von 37 weiteren Patienten wurde analysiert. Zu Beginn der Therapie lag bei etwa 51% der Patienten (n=19) ein Vitamin D Mangel [25(OH)VitD - Serumspiegel < 30ng/ml Serum bzw. 75 nmol/l] vor. Bei 27% der Patienten (n=10) lag der 25(OH)VitD - Serumspiegel unter 20ng/ml Serum bzw. 50 nmol/l (=schwerer Vitamin D Mangel). Die folgende Tabelle 2 zeigt in der jeweils linken Spalte die tägliche Aufnahme an Kalzium durch die Nahrung im mg. Die tägliche Zufuhr von weniger als 1000mg durch Nahrungsmittel führt zu einer negativen Kalziumbilanz. Daraus entwickelt sich ein sekundärer Hyperparathyreodismus und Knochensubstanz geht verloren. Eine tägliche Versorgung von weniger als 500mg Kalzium ist mit einem erhöhten Frakturrisiko für nicht-vertebrale Frakturen verbunden.

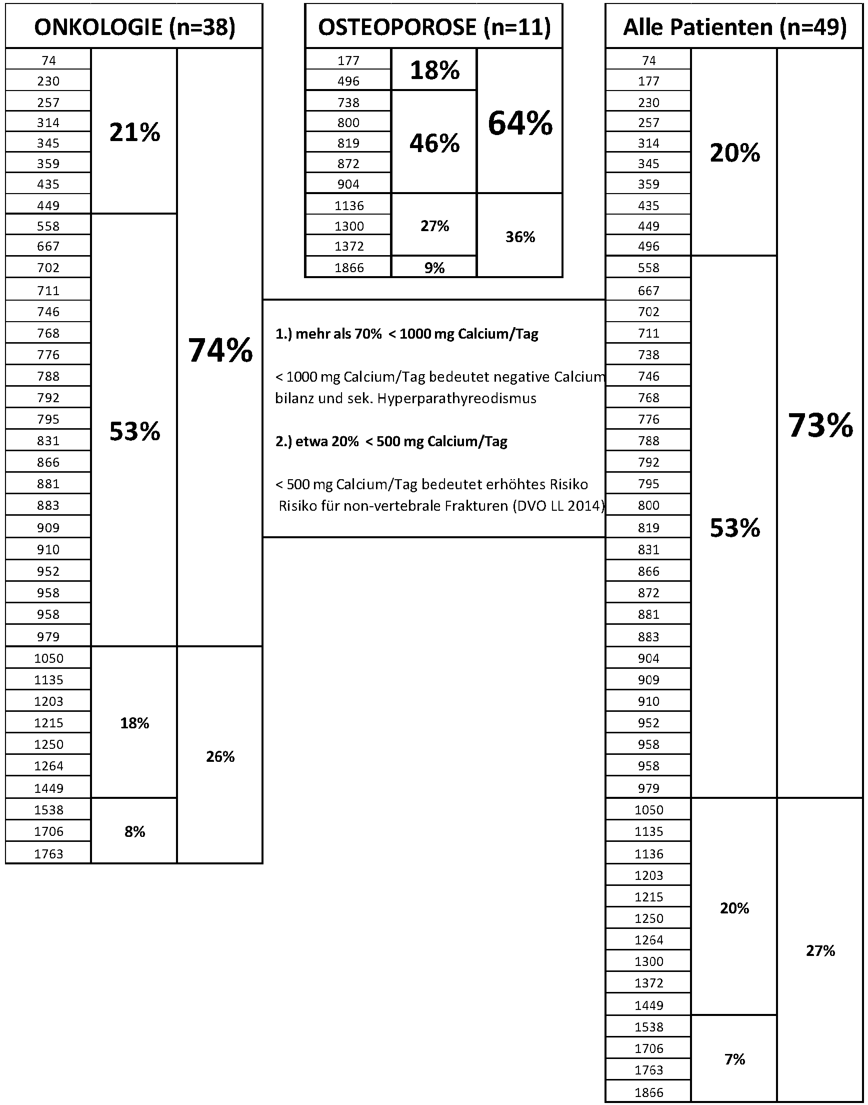

### Beispiel 3:

Bei 3 Patienten wurde der sogenannte "Chair-Rising-Test" (Aufstehtest) durchgeführt.

Der Chair-Rising-Test (Aufstehtest) lässt eine Aussage über die Kraftverhältnisse und das Sturzrisiko der Testperson zu. Die Testperson wird aufgefordert, so schnell wie möglich mit vor der Brust verschränkten Armen fünfmal von einem Stuhl üblicher Höhe (ca. 46 cm Sitzhöhe) ohne Einsatz der Arme aufzustehen und sich wieder hinzusetzen. Gelingt dies nicht 5x hintereinander, so werden nicht die Sekunden notiert, sondern die Anzahl der gelungenen Durchführungen. Werden mehr als 10-11 Sekunden benötigt, muss man von einer erhöhten Sturzgefahr ausgehen.

**Tabelle 3: Der Chair-Rising-Test wurde bei drei Osteoporose-Patienten vor Beginn der Therapie mit 60 mg Anti-RANKL-Antikörper/Denosumab (Prolia) + 500 mg Calcium + 1000 IE Vitamin D3 durchgeführt (linke Spalte) und 6 Monate später wiederholt (mittlere Spalte). Die Verbesserung ist in der rechten Spalte in % angegeben.**

| Chair-Rising | Chair-Rising | Verbesserung |
|---|---|---|
| 9 sec | 8 sec | + 11% |
| 10 sec | 8 sec | + 20% |
| 14 sec | 10 sec | + 29% |

Die Sturzgefahr konnte durch die Therapie zwischen 11-29% verringert werden.

### Beispiel 4:

Die folgenden Daten von einem onkologischen Patienten (Prostatakarzinom) in Tabelle 4 zeigen die Serumwerte von Kalzium und Vitamin D3 bei Verabreichung von 120mg Denosumab (Anti-RANKL-Antikörper, Xgeva) 4-wöchentlich, sowie der oralen Einnahme von 500 mg Kalzium täglich und 1000 I.E. Vitamin D3 täglich.

**Tabelle 4: Der Patient erhielten 120 mg Denosumab 4-wöchentlich und 500mg Kalzium und 1000 I.E. Vitamin D3:**

| Patient/-in | Kalzium mmol/l | 25(OH) Vitamin D3-Serumspiegel; µg/l | nach Wochen | Kalzium mmol/l | 25(OH) Vitamin D3-Serumspiegel; µg/l | Kalzium in Ernährung mg/Tag |
|---|---|---|---|---|---|---|
| 1 | 2,01 | 36,7 | 13/26 | 2,11/2,12 | 28,3/33,5 | 883 |

Die folgenden Daten von Osteoporose-Patienten in Tabelle 5 zeigen die Serumwerte von Kalzium und Vitamin D3 bei Verabreichung von halbjährlich 60mg Anti-RANKL-Antikörper/Denosumab (Prolia) sowie der oralen Einnahme von 500 mg Kalzium täglich und 1000 I.E. Vitamin D3 täglich.

**Tabelle 5: Die Osteoporose-Patienten erhielten halbjährlich 60 mg Denosumab, täglich 500mg Kalzium und ebenfalls täglich 1000 I.E. Vitamin D3:**

| Patient/-in | Kalzium mmol/l | 25(OH) Vitamin D3-Serumspiegel; µg/l | nach Wochen | Kalzium mmol/l | 25(OH) Vitamin D3-Serumspiegel; µg/l | Kalzium in Ernährung mg/Tag |
|---|---|---|---|---|---|---|
| 2 | 2,32 | 40,8 | 13/26 | 2,37/2,45 | 50,8/44,1 | 904 |
| 3 | 2,21 | 41,9 | 26 | 2,32 | 31,5 | 1300 |
| 4 | 2,33 | 50 | 13/26 | 2,34/2,32 | 58,1/31,5 | 1136 |
| 5 | 2,31 | 29,2 | 13 | 2,32 | 24,8 | 1372 |
| 6 | 2,39 | 36 | 13 | 2,3 | 29,6 | 819 |

Bei einem 25(OH) Vitamin D - Serumspiegel < 20ng/ml liegt ein schwerer Vitamin D-Mangel vor, der nach der DVO-Leitlinie ein erhöhtes Frakturrisiko bedeutet. Die Daten in Beispiel 4 zeigen, dass die erfindungsgemäße Kombination geeignet ist, den 25(OH)Vitamin D-Spiegel innerhalb von 3-6 Monaten über 20ng/ml bzw. 20µg/l zu normalisieren und dadurch das Frakturrisiko zu senken.

Der Kalzium-Serumspiegel ist bei Patienten mit Osteoporose in der Regel im Normbereich. Bei onkologischen Patienten kann die Kalziumkonzentration im Blut ansteigen (=tumorinduzierte Hyperkalzämie) oder therapiebedingt absinken (therapie-induzierte Hypokalzämie). Patienten mit einer kalziumarmen Ernährung haben deshalb ein zusätzliches Hypokalzämie-Risiko. Eine erfindungsgemäße tägliche Supplementation mit 500 mg Kalzium sogt für eine ausgeglichene Kalziumbilanz.

Ein erhöhter PTH-Spiegel (=sekundärer Hyperparathyreodisumus) sowie eine Hypokalzämie - induziert durch eine anti-resorptive Therapie mit Anti-RANKL-Antikörpern (Denosumab) - sind Risikofaktoren für die Entstehung einer Nekrose am Kieferknochen. Aus diesem Grund verringert die erfindungsgemäße Supplementation mit Kalzium und Vitamin D das Risiko für Kiefernekrosen.

Die Daten zeigen, dass die erfindungsgemäße Kombination geeignet ist, den 25(OH)Vitamin D-Serumspiegel innerhalb von 3-6- Monaten über 20ng/ml bzw. 20µg/l zu normalisieren und bei nahezu allen Patienten eine positive Kalziumbilanz zu erzeugen. Die Patienten waren frei von Nebenwirkungen, insbesondere traten keine Kieferosteonekrosen, nicht heilende Wunden bis zur Auflösung des Kiefers, therapiebedingten Hypokalzämien, kardiovaskuläre Nebenwirkungen wie Herzinfarkt, Vorhofflimmern, Herzrhythmusstörungen, sekundärer Hyperparathyreodismus, Krämpfe und/oder Taubheitsgefühl auf.

Es traten ferner keine skelettbezogenen Komplikationen, insbesondere aufgrund von soliden Tumoren, vorzugsweise Mamma-, Prostata-, Lungen-, Darm- oder Knochenkarzinom (Osteosarkom) auf, keine (pathologischen) Frakturen, Bestrahlung des Knochens, Rückenmarkskompression oder operative Eingriffe am Knochen, Knochenmetastasen, Schmerzen bei Knochenmetastasen, Nerveneinklemmungen, Deformierungen aufgrund einer oder mehrerer solider Tumore wie beispielsweise Brustkrebs, Prostatakrebs, Lungenkrebs oder multiplem Myelom.

Ferner bezieht sich die Erfindung auf die folgenden Gegenstände:
1. Verwendung von pharmazeutischen Zusammensetzungen umfassend Anti-RANKL-Antikörper und Kalzium und Vitamin D zur Behandlung von Osteoporose, postmenopausaler Osteoporose, manifester Osteoporose, corticoinduzierter Osteoporose, Osteoporose des Mannes oder der Frau, Morbus Paget, Osteogenesis imperfecta, zur Prophylaxe von Frakturen der vorgenannten Erkrankungen, sowie zur Verwendung in der Behandlung und/oder Prophylaxe der durch Anti-RANKL-Antikörper therapiebedingten Hypokalzämie, dadurch gekennzeichnet, dass die pharmazeutische Zusammensetzung umfassend Anti-RANKL-Antikörper verabreicht wird, vorzugsweise in einer Dosierung von 30 bis 90 mg in 1-2 ml Lösung (20-60 mg/ml), stärker bevorzugt 50-70 mg, noch stärker bevorzugt 60mg, bevorzugt in 1 ml Lösung (60 mg/ml) subkutan jährlich oder halbjährlich und die pharmazeutische(n) Zusammensetzung(en) umfassend Kalzium mit einer Dosierung von 400-600mg pro Tag, besonders bevorzugt 500mg pro Tag oral verabreicht wird und Vitamin D in einer Dosierung von 800-1200 I.E. Vitamin D, besonders bevorzugt 1000 I.E. Vitamin D täglich oral verabreicht wird.
2. Verwendung von pharmazeutischen Zusammensetzungen umfassend Anti-RANKL-Antikörper und Kalzium und Vitamin D zur Behandlung und/oder Prophylaxe skelettbezogener Komplikationen, insbesondere aufgrund von soliden Tumoren, vorzugsweise Mamma-, Prostata-, Lungen-, Darm- oder Knochenkarzinom (Osteosarkom), pathologischen Frakturen, Bestrahlung des Knochens, Rückenmarkskompression oder operativen Eingriffen am Knochen, Knochenmetastasen, Schmerzen bei Knochenmetastasen, Nerveneinklemmungen, Deformierungen aufgrund eines oder mehrerer solider Tumore wie beispielsweise Brustkrebs, Prostatakrebs, Lungenkrebs oder multiplem Myelom, sowie der Prophylaxe von Frakturen bei den vorgenannten Erkrankungen und zur Verwendung in der Behandlung und/oder Prophylaxe der durch Anti-RANKL-Antikörper therapiebedingten Hypokalzämie, dadurch gekennzeichnet, dass Anti-RANKL-Antikörper verabreicht werden, vorzugsweise in einer Dosierung von 60-180 mg in 1-3 ml Lösung, stärker bevorzugt 80-150 mg, noch stärker bevorzugt 120 mg, insbesondere in 1,7 ml Lösung (70 mg/ml) zur mindestens 3-4 wöchigen subkutanen Anwendung und 400-600mg Kalzium täglich, besonders bevorzugt 500mg Kalzium täglich und 800-1200 I.E. Vitamin D täglich, besonders bevorzugt 1000 I.E. Vitamin D täglich oral verabreicht werden.
3. Verwendung von pharmazeutischen Zusammensetzungen nach Gegnstand 1-2, wobei die pahrmazeutische Zusammensetzung geeignet ist, zur Behandlung und/oder Prophylaxe von Nebenwirkungen der Anti-RANKL-Antikörper, insbesondere Kieferosteonekrosen, nicht heilenden Wunden bis zur Auflösung des Kiefers, Hypokalzämien, kardiovaskulären Nebenwirkungen wie Herzrhythmusstörungen, Krämpfe, und sekundärer Hyperparathyreodismus, verwendet zu werden.
4. Verwendung von pharmazeutischen Zusammensetzungen nach Gegenständen 1-3, dadurch gekennzeichnet, dass der Anti-RANKL-Antikörper eine schwere Kette und eine leichte Kette umfasst, wobei die schwere Kette eine Aminosäuresequenz nach SEQ ID NO: 2 oder SEQ ID NO: 8 und die leichte Kette eine Aminosäuresequenz nach SEQ ID NO: 4 oder SEQ ID NO: 9 umfasst.
5. Verwendung von pharmazeutischen Zusammensetzungen nach Gegenständen 1-4, dadurch gekennzeichnet, dass der Anti-RANKL-Antikörper die variable Region der schweren Kette die Aminosäuresequenz nach SE ID NO: 5 und/oder die variable Region der leichten Kette die Aminosäuresequenz nach SEQ ID NO: 6 umfasst.
6. Verwendung von pharmazeutischen Zusammensetzungen nach Gegenstand 1-5, wobei der Anti-RANKL-Antikörper ein monoklonaler, polyklonaler, monospezifischer, bispezifischer, bifunktionaler, einzelkettiger, synthetischer, rekombinanter, mutierter, humaner, humanisierter, chimärer, IgG, IgA, IgM oder IgE Antikörper, ein antigenbindendes Fragement oder ein Antikörper-Konstrukt wie Fvs (scFv) aus Einzelketten oder Antikörper-Fusionsproteinen bzw. ein Fragment davon, insbesondere getrennte leichte und schwere Ketten, Fab, Fab/c, Fv, scFv, Fd, dAb, Fab' oder F(ab')2 ist, wobei ein Fragment eine variable Region der leichten Kette und/oder eine variable Region der schweren Kette umfasst.
7. Verwendung von pharmazeutischen Zusammensetzungen nach Gegenstand 1-6, wobei Kalzium insbesondere Kalziumlaktatgluconat, Kalziumgluconat-Monohydrat, Kalziumlaktat-Pentahydrat, Kalziumzitrat, Kalziumzitrat-Tetrahydrat, Kalziumkarbonat, Kalzium-Phosphat, Kalzium-Dihydrogenphosphat, Kalzium-Hydrogenphosphat, Kalzium-Hydrogenphosphat Dihydrat, Kalzium-Acetat, Kalzium-Ascorbat, Kalzium-Chloride, Kalzium-Glucoheptonat, Kalzium-Glycerophosphat und/oder Kalzium-Sulfat umfasst.
8. Verwendung von pharmazeutischen Zusammensetzungen nach Gegenstand 1-7, wobei Vitamin D insbesondere Vitamin D und/oder Vitamin D3 und/oder Vitamin D2 und/oder deren Derivate, insbesondere Calcitriol (1,25-Dihydroxyvitamin D3) oder 1α, 25-Dihydroxycholecalciferol, Alphacalcidol (1α-Hydroxyvitamin D3), 24,25-Dihydroxyvitamin D3, Calcifediol, Vitamin D2 und/oder Ergocalciferol umfasst.
9. Pharmazeutische Zusammensetzungen nach den Gegenständen 1-8, zusätzlich umfassend pharmazeutisch geeignete Hilfsstoffe und/oder Flüssigkeiten oder Lösungsmittel.
10. Verwendung von pharmazeutischen Zusammensetzungen nach den Gegenständen 1-9, wobei die Hilfsstoffe für die pharmazeutischen Zusammensetzungen Lactose, Stärke, Säuerungsmittel insbesondere Citronensäure, Apfelsäure, Säureregulatoren insbesondere Natriumhydrogencarbonat und Natriumcarbonat, Feuchthaltemitteln insbesondere Sorbitol, Xylitol und Inulin, Trennmitteln insbesondere Tricalciumphosphat, Speisefettsäuren insbesondere Magnesiumsalze insbesondere Magnesiumstearat, natürliche und naturidentische und andere Aromen und Aromastoffe, Süßstoffe insbesondere Natrium-Cyclamat, Aspartam und Saccharin-Natrium, Maltodextrin, Farbstoffe insbesondere Rote-Bete-Saftpulver und Riboflavin-5'-phosphat, Siliciumdioxid insbesondere hochdispers, Siliciumdioxid hydrat, Phenylalanin, Arabisches Gummi, Saccharose, Gelatine, Maisstärke, Sojaöl, Glycerol, DL-alpha-Tocopherol, Isomalt, Natriumhydrogencarbonat, Natriumdihydrogencarbonat, Natriumcitrat, Natriumdihydrogencitrat, Carmellose Natrium, Acesulfam Kalium, Natriumascorbat, Triglyceride insbesondere mittelkettige, und/oder in pharmazeutisch verträglichen Flüssigkeiten, insbesondere Wasser, isotonischer Kochsalzlösung, isotonischer Sorbitol-Natriumacetat-Polysorbat-Lösung, isotonischer Sorbitol-Natriumaceat-Lösung, isotonischer Glucoselösung oder anderen pharmazeutisch geeigneten isotonischen Lösungen geeigneten pH-Werts umfassen.
11. Verwendung von pharmazeutischen Zusammensetzungen nach den Gegenständen 1-10, wobei die Lösungsmittel oder Flüssigkeiten Wasser, isotonische Kochsalz- oder Glucoselösung umfassen.
12. Verwendung von pharmazeutischen Zusammensetzungen nach den Gegenständen 1-11, wobei die pharmazeutische Zusammensetzung umfassend Anti-RANKL-Antikörper als Infusion, Infusionslösungskonzentrat, Fertiginfusion oder Fertigspritze und die pharmazeutischen Zusammensetzungen umfassend Kalzium und/oder Vitamin D in fester oder flüssiger Form als Brausetablette, Schlucktablette oder -kapsel, Kautablette, Brausegranulat, Direktgranulat, Trinklösung, Tropfen, Sublingualspray, einzeln oder zusammen bereitgestellt wird.

## Patentansprüche

1. Anti-RANKL-Antikörper und Kalzium und Vitamin D3 zur Verwendung bei der Behandlung und/oder Prophylaxe der durch Anti-RANKL-Antikörper bedingten Hypokalzämie, wenn die Anti-RANKL-Antikörper bei skelettbezogenen Komplikationen, aufgrund von Mamma-, Prostata-, Lungen-, Darm- oder Knochenkarzinom (Osteosarkom) oder multiplem Myelom angewendet werden, **dadurch gekennzeichnet, dass**
a) Anti-RANKL-Antikörper in einer Dosierung von 60-180 mg in 1-3 ml Lösung oder 80-150 mg in 1-3 ml Lösung oder 120 mg in 1-3 ml Lösung oder in 1,7 ml Lösung (70 mg/ml) jeweils mindestens 3-4 Wochen als subkutane Anwendung und
b) 400-600 mg Kalzium täglich oder 500 mg Kalzium täglich oral und
c) 800-1200 I.E. Vitamin D3 täglich oder 1000 I.E. Vitamin D3 täglich oral verabreicht werden, **dadurch gekennzeichnet, dass** eine pharmazeutische Zusammensetzung umfassend die Anti-RANKL-Antikörper als Infusion, Infusionslösungskonzentrat, Fertiginfusion oder Fertigspritze verwendet wird und Kalzium und Vitamin D3 als Kautablette oder Direktgranulat verwendet wird.

2. Anti-RANKL-Antikörper und Kalzium und Vitamin D3 zur Verwendung nach Anspruch 1, **dadurch gekennzeichnet, dass** der Anti-RANKL-Antikörper eine schwere Kette und eine leichte Kette umfasst, wobei die schwere Kette eine Aminosäuresequenz nach SEQ ID NO: 2 oder SEQ ID NO: 8 und die leichte Kette eine Aminosäuresequenz nach SEQ ID NO: 4 oder SEQ ID NO: 9 umfasst.

3. Anti-RANKL-Antikörper und Kalzium und Vitamin D3 zur Verwendung nach Ansprüchen 1-2, **dadurch gekennzeichnet, dass** der Anti-RANKL-Antikörper die variable Region der schweren Kette die Aminosäuresequenz nach SEQ ID NO: 5 und/oder die variable Region der leichten Kette die Aminosäuresequenz nach SEQ ID NO: 6 umfasst.

4. Anti-RANKL-Antikörper und Kalzium und Vitamin D3 zur Verwendung nach Anspruch 1-3, wobei der Anti-RANKL-Antikörper ein monoklonaler, polyklonaler, monospezifischer, bispezifischer, bifunktionaler, einzelkettiger, synthetischer, rekombinanter, mutierter, humaner, humanisierter, chimarer, IgG, IgA, IgM oder IgE Antikörper, ein antigenbindendes Fragement oder ein Antikörper-Konstrukt wie Fvs (scFv) aus Einzelketten oder Antikörper-Fusionsproteinen bzw. ein Fragment davon, insbesondere getrennte leichte und schwere Ketten, Fab, Fab/c, Fv, scFv, Fd, dAb, Fab' oder F(ab')2 ist, wobei ein Fragment eine variable Region der leichten Kette und/oder eine variable Region der schweren Kette umfasst.

5. Anti-RANKL-Antikörper und Kalzium und Vitamin D3 zur Verwendung nach Anspruch 1-4, wobei Kalzium insbesondere Kalziumlaktatgluconat, Kalziumgluconat-Monohydrat, Kalziumlaktat-Pentahydrat, Kalziumzitrat, Kalziumzitrat-Tetrahydrat, Kalziumkarbonat, Kalzium-Phosphat, Kalzium-Dihydrogenphosphat, Kalzium-Hydrogenphosphat, Kalzium-Hydrogenphosphat Dihydrat, Kalzium-Acetat, Kalzium-Ascorbat, Kalziumchlorid, Kalzium-Glucoheptonat, Kalzium-Glycerophosphat und/oder Kalzium-Sulfat umfasst.

6. Anti-RANKL-Antikörper und Kalzium und Vitamin D3 zur Verwendung nach den Ansprüchen 1-5, zusätzlich umfassend pharmazeutisch geeignete Hilfsstoffe und/oder Flüssigkeiten oder Lösungsmittel.

7. Anti-RANKL-Antikörper und Kalzium und Vitamin D3 zur Verwendung nach den Ansprüchen 1-6, wobei als Hilfsstoffe Lactose, Stärke, Säuerungsmittel insbesondere Citronensäure, Apfelsäure, Säureregulatoren insbesondere Natriumhydrogencarbonat und Natriumcarbonat, Feuchthaltemitteln insbesondere Sorbitol, Xylitol und Inulin, Trennmitteln insbesondere Tricalciumphosphat, Speisefettsäuren insbesondere Magnesiumsalze insbesondere Magnesiumstearat, natürliche und naturidentische und andere Aromen und Aromastoffe, Süßstoffe insbesondere Natrium-Cyclamat, Aspartam und Saccharin-Natrium, Maltodextrin, Farbstoffe insbesondere Rote-Bete-Saftpulver und Riboflavin-5'-phosphat, Siliciumdioxid insbesondere hochdispers, Siliciumdioxid hydrat, Phenylalanin, Arabisches Gummi, Saccharose, Gelatine, Maisstärke, Sojaöl, Glycerol, DL-alpha-Tocopherol, Isomalt, Natriumhydrogencarbonat, Natriumdihydrogencarbonat, Natriumcitrat, Natriumdihydrogencitrat, Carmellose Natrium, Acesulfam Kalium, Natriumascorbat, Triglyceride insbesondere mittelkettige, und/oder in pharmazeutisch verträglichen Flüssigkeiten, insbesondere Wasser, isotonischer Kochsalzlösung, isotonischer Sorbitol-Natriumacetat-Polysorbat-Lösung, isotonischer Sorbitol-Natriumacetat-Lösung, isotonischer Glucoselösung oder anderen pharmazeutisch geeigneten isotonischen Lösungen geeigneten pH-Werts verwendet werden.

8. Anti-RANKL-Antikörper und Kalzium und Vitamin D3 zur Verwendung nach den Ansprüchen 6-7, wobei die Lösungsmittel oder Flüssigkeiten Wasser, isotonische Kochsalz-oder Glucoselösung umfassen.

9. Anti-RANKL-Antikörper und Kalzium und Vitamin D3 zur Verwendung nach wenigstens einem der Ansprüche 1 bis 8, worin Kalzium und Vitamin D3 als Direktgranulat bereitgestellt werden.

10. Anti-RANKL-Antikörper und Kalzium und Vitamin D3 zur Verwendung nach wenigstens einem der Ansprüche 1 bis 9, worin
a) die Anti-RANKL-Antikörper in einer Dosierung von 120 mg in 1-3 ml Lösung oder in 1,7 ml Lösung (70 mg/ml) verabreicht werden,
b) das Kalzium in einer Dosierung von 500 mg und
c) das Vitamin D3 in einer Dosierung von 1000 I.E.
verabreicht werden.
